# EUROPEAN PATENT SPECIFICATION

(11) **EP 1 970 450 B1**
(45) Date of publication and mention of the grant of the patent: **17.11.2010**
(21) Application number: 06846928.7
(22) Date of filing: 19.12.2006
(51) Int. Cl.: C12Q 1/04, C12N 1/16, C12R 1/72

(54) **NUTRIENT MEDIUM FOR YEAST CULTURE**
NÄHRMEDIUM FÜR HEFEKULTUR
MILIEU NUTRITIF POUR LA CULTURE DE LEVURES

(30) Priority: 19.12.2005 CU 2702005; 05.12.2006 CU 2382006
(43) Date of publication of application: 17.09.2008
(73) Proprietor: CENTRO NACIONAL DE BIOPREPARADOS, Bejucal, Provincia La Habana 6048 (CU)
(72) Inventor: LOBAINA RODRÍGUEZ, Tamara, Bejucal, La Habana 6048 (CU); RODRÍGUEZ MARTINEZ, Claudio, Bejucal, La Habana 6048 (CU); ZHURBENKO, Raisa, Bejucal, La Habana 6048 (CU); GARCÍA MARICHAL, José Miguel, Bejucal, La Habana 6048 (CU); ZAYAS RUIZ, Yordania, Bejucal, La Habana 6048 (CU)
(74) Representative: Gil-Vega, Victor
(86) International application number: PCT/CU2006/000016
(87) International publication number: WO 2007/071204

(56) References cited:
- EP-A1- 0 544 605
- WO-A1-00/50610
- WO-A1-91/14787
- US-B1- 6 921 635
- SAKAKIBARA MITSUTAKA: "Development of a meridic diet for adults of the West Indian sweetpotato weevil, Euscepes postfasciatus (Fairmaire) (Coleoptera: Curculionidae)." JAPANESE JOURNAL OF APPLIED ENTOMOLOGY AND ZOOLOGY, vol. 47, no. 2, 2003, pages 67-72, XP002530745 ISSN: 0021-4914
- DATABASE CAPLUS [Online] BAE J.S. ET AL.: 'studies of the Production of Fermented Feeds. 2. Conditions of Enzyme Production and Influence of Addition of Ammonium Sulfate in the Culture of Useful Yeasts', XP003014776 Database accession no. (1975:15222) & MISAENGMUL HAKHOECHI vol. 9, no. 1, 1971, pages 32 - 38
- PERRY J.L. ET AL.: 'Umbelliferyl-Labeled Glactosaminide as an Aid in Identification of Candida albicans' J. CLIN. MICROBIOL. vol. 25, no. 12, 1987, pages 2424 - 2425, XP003014786

## Description

### OBJECT AND BACKGROUND OF THE INVENTION

This invention relates to the field of microbiologic diagnosis, specifically to culture and identification of yeasts of clinical relevance, in particular, of diverse species of the genus *Candida.*

The United States patent No. 4144133 discloses the use of ox bile, purified saponin and a substrate for detection of the enzyme phenol oxidase, which facilitates the identification of pathogenic fungi, specifically *Candida albicans* and *Cryptococcus neoformans.* However, this method does not provide for growth, identification and differentiation of all *Candida* species of clinical or environmental relevance.

Komatsu Osamu, in Japanese patent JP2003310298, describes a method to discriminate *Candida albicans* and *Candida tropicalis* against other fungi by using X-glc substrate. The specificity of this substrate does not guarantee appropriate differentiation of all *Candida* species of clinical and/or environmental relevance.

Beckford, O. A. and collaborators, in patent No. 4030980, described an apparatus for isolation and identification of yeasts of clinical origin by using several biochemical tests and culture media for identification of *Candida* species, among other microorganisms. The apparatus and the technique for its employment have the following essential inconveniences: the complexity of production of such device, the complexity involved in the inoculation into each of the dishes and the necessity of isolation of the colonies, among other disadvantages. The most outstanding inconveniences are that the dishes for biochemical tests do not contain sufficient, appropriate nutrients for the growth of *Candida* species, and the identification is carried out by observing multiple biochemical responses, therefore an atypical response in one of the dishes can lead to an erroneous identification.

The British patent GB 730763 describes a culture medium for isolation and identification of species of the genus *Candida,* the formulation of which includes an inorganic bismuth salt and sodium sulfite, together with a solidification agent. This medium includes a soluble carbohydrate, sodium piruvate, glycine, organic phosphate and yeast extract. This culture medium has a number of technical inconveniences, namely:
- It does not supply all the nutrients that are necessary to ensure abundant, quick growth of all relevant *Candida* species, since it contains a single source of vitamins, specifically yeast extract, the content of minerals of which, of magnesium in particular, is insufficient for a significant activation of the complex enzymatic system of these microorganisms.
- Likewise, the medium contains a single energy source -sodium piruvate- which is insufficient to ensure uniform, quick, abundant growth of the diverse *Candida* species.
- The medium does not provide for differentiation of the diverse *Candida* species from each other.
- Glycine alone cannot provide sufficient nitrogen for the synthesis of amino acids, proteins and enzymes required for quick and exuberant growth of *Candida* species.

Jesús Ruiz-Aragón and collaborators (Assessment of a new CHROMOagar Candida medium for presumptive identification of yeasts; Revista de Diagnóstico Biológico. V.52(1), 2003) compared the new medium CHROM M with the media CHROM OR and CHROM R (Beckton Dickinson). Table 1 of that paper shows that the CHROM OR medium cannot differentiate relevant *Candida* species from each other. In the case of CHROM R, not all relevant species can be thoroughly differentiated, while in the medium CHROM M several species showed equal or similar coloration. The authors highlight that the results can be better appreciated only after 48 hours of incubation. Also, they concluded that CHROM M did not prove to be equally effective for identification of diverse yeast species, since it is less sensitive, needs greater time of incubation for development of the colonies, and hinders the differentiation of some species of clinical relevance such as *Candida tropicalis* and *Candida parapsilosis.*

The United States patent No. 5449612 describes a method for identification of *Candida albicans* and *Torupsis glabrata* strains by using chromogenic substrates of monoamino acids or peptides without need of isolating the strain to be identified. The composition of the medium incorporates cycloheximide to inhibit the growth of some *Candida* species other than *Candida albicans*, an inclusion that limits the diagnostic possibilities of this variant of the medium. The addition of antibacterial substances such as gentamicin decreases the stability of the medium upon its preparation; therefore it cannot be conserved for a prolonged time. Chloramphenicol does also interfere with the development of chromogenic reactions.

Furthermore, the nutritional bases employed as nitrogen sources in that invention (Yeast Nitrogen Bases, Neopeptone, Proteose Peptone, Bacto Peptone and Yeast Extract) alone or in their combinations cannot provide sufficient contents of vitamins and minerals, macro- and microelements for early growth of *Candida* species.

The United States patent No. 5534415 describes a selective differential culture medium for growth and detection of *Candida albicans*. The essential element of the culture medium lies on its having a nutritional base to be metabolized by yeasts, a chromogenic or fluorogenic substrate that can be hydrolyzed by the enzyme hexosaminidase associated to *Candida albicans* and at least a compound containing hexosaminidase, which activates the enzyme hexosaminidase. The components acting as a nitrogen source for the medium are peptones in quantities ranging from 0.01 to 40 g/L; such are, for example, meat peptone, soy peptone and peptone mixtures; also yeast extract, in a quantity ranging from 0.01 to 40 g/L. These nitrogen compounds, by themselves alone, do not provide all the nutrients and vitamins necessary for an abundant, early growth of *Candida* species; also, they do not ensure well defined chromogenic reactions with intense coloration during the early hours of growth of *Candida* species.

The inclusion in the formulation of carbon sources at the concentrations indicated by the authors, such as glucose, acetate, lactate, aminobutyrate, etc. or their mixtures can only be employed in combination with this specific type of chromogenic substrate, since it would compete with the enzymatic activity of *Candida* species and would limit, in turn, the possibility to incorporate other chromogenic or fluorogenic substrates to identify or differentiate other *Candida* species. Such is the case of glucose, which would impede or weaken glucosidase activity. The composition of the medium includes such buffers as phosphates, TRIS, HEPES and citrates in such quantities that they adjust the pH close to 7, thereby favoring *Candida albicans* only, but not other species of the genus *Candida* of sanitary or environmental relevance.

Other activators may be added to the medium, such as magnesium, manganese and/or calcium salts in quantities ranging from 0 to 10 mMol/L.

The formulation includes cell permeability enhancers such as bile salts, sodium deoxycholate, polyoxyethyleneglycol, alkyl phenol ethers, sorbitan or fatty acid esters at a concentration ranging from 0 to 5 g/L. Some of these substances, bile salts for example, are extremely growth-inhibiting to *Candida* species, even to *Candida albicans*.

The medium is also added a mixture of Gram-positive and Gram-negative bacteria inhibitors, such as gentamicin, penicillin, etc. Most of these substances are unstable to heat and should be prepared and used quickly. Also, the prepared media ready to be used in Petri dishes should be stored in refrigeration only for not too long conservation periods ranging from 2 to 30 days.

A description is made of the possibility of employing tellurite, molybdate or their mixtures, which have likewise high growth-inhibiting effects on *Candida* species.

Among other evident disadvantages of the diverse media described in the invention, worthwhile to be mentioned is that, even after 24 hours of incubation, not all the colonies of *Candida albicans* had developed a strong coloration. Over 48 hours were required for most of *Candida albicans* colonies to give an intense color response, whereas other *Candida* species did not give color response at all (Tables II and III in original work).

Several authors have reported the use of vegetable extracts for different purposes to enhance growth or differentiation of *Candida* species.

Zia U. Khan and collaborators (Tobacco Agar, a New Medium for Differentiating Candida dubliniensis from Candida albicans, J. Clin. Microb., v. 42(10):4796-4798, 2004) used sunflower seed agar in cultures of *Candida dubliniensis*. They likewise described the use of tobacco leave extract and nicotine in a culture medium. None of the extracts used can provide sufficient, appropriate vitamins and sugars for the growth of relevant *Candida* species. Neither, the media referred to above do not provide for differentiation of all the species from each other. Lastly, differentiation and identification are not only based on dissimilar colors, but also on the morphological characteristics of the colonies based on chlamydospore production, therefore, skill personnel are required for interpretation of the results.

Dostál and collaborators, 2003, described the employment of a culture medium containing bovine hemoglobin, yeast extract and bromophenol blue to detect proteolytic activity of *Candida.* (Dostál J., Hamal P., Pavlicková L., Soucek M., Ruml T., Pichova I., Huskova-H O. Simple Method for Screening Candida Species Isolates for the Presence of Secreted Proteinases: A Tool for the Prediction of Successful Inhibitory Treatment. J. Clin. Microbiol. 2003, Feb., 41 (2):712-716). The essential inconveniences lie on the fact that hemoglobin detects only the asparto-protease activity present in all the species, hence it is useless to differentiate them from each other. Also, it is necessary to filter the hemoglobin and add it as a supplement under aseptic conditions, a requirement that hinders the procedure. Other deficiencies are the necessity of dissolving the indicator in ethanol, the excessively low pH of the medium (4 to 4.5), not optimal to promote growth of yeasts, the incubation temperature of 30 degrees (not optimal also) and that the detection of the proteolytic activity can take from 5 to 7 days.

Bramono K. and collaborators, 2006, studied diverse enzymatic activities in *Candida* species, specifically proteinase, lipase and α-glucosidase in minimal media which were faulty in nutrients. (Bramono K., Yamazaki M., Tsuboi R. and Ogawa H. Comparison of Proteinase, Lipase and α-Glucosidase Activities from the Clinical Isolates of Candida Species. Jpn. J. Infect. Dis., v. 59, 73-76, 2006). The authors found significant extracellular proteinase activity in *Candida albicans, Candida parapsilosis* and *Candida tropicalis.* However, this activity was linked to protein hydrolysis of bovine serum (albumin) as the source of nutrients for growth and it did not play any role as identification or differentiation tool, since this activity is also present in other species, albeit on a lower degree. Albumin addition as marker of proteolytic activity reduces stability of the medium. Lipolytic activity was induced by using Tween 80, the only carbon source used in this medium. This activity was significantly greater in *Candida tropicalis, Candida albicans* and *Candida parapsilosis.*

The α-glucosidase activity was tested by adding 0.6 % maltose to a minimal medium and using p-nitrophenyl-α-D-glucopyranoside. The optical density was read on a spectrophotometer. The results proved that *Candida tropicalis, Candida albicans* and *Candida parapsilosis* exert the greatest enzymatic activity in a liquid minimal medium. The reaction is not appreciable at naked eye, but it is necessary to use laboratory equipment for the reading and interpretation of results.

The objective of the abovementioned work was to carry out basic studies on *Candida* enzymatic activity under conditions of low nutritional level, without any pretension of species identification or differentiation.

Asmaa Al Mosaid and collaborators (Differentiation of Candida dubliniensis from Candida albicans on Staib Agar and Caffeic Citrate Agar, J. Clin. Microb., v. 39(1), 323-327, 2001), described the identification of the pathogenic *Candida dubliniensis* in two culture media. In one of them a watery extract of *Guizotia abyssinica* seeds was used with addition of glucose and creatinine. The other one used yeast extract, glucose, ammonium sulfate, ferric citrate and caffeic acid. None of the abovementioned media can ensure early growth of *Candida,* mainly due to the fact that they lack sufficient contents of vitamins, nitrogen substances and carbohydrates to promote quick and abundant growth of all *Candida* species.

The same author, in his paper "Differentiation of Candida dubliniensis from Candida albicans on Pal's Agar" (J. Clin. Microb., v. 41(10): 4787-4789, 2003), compared the employment of Pal's agar by using desalinized extract of sunflower seeds with the Staib agar. In both cases, identification and differentiation of *Candida* were achieved upon 48-72 hours of incubation. This work leads to the same conclusions previously mentioned with regard to the deficiencies of the media compared.

Venitia M. C. and collaborators (New Chromogenic Agar Medium for the Identification of Candida spp. J. of Virology, v.68(7): 3622-3627, 2002) described a new culture medium for identification of *Candida* species based on the use of Sabouraud's Dextrose Agar with addition of a chromogenic substrate for detection of glucosaminidase activity of *Candida albicans* and *Candida dubliniensis.* This medium was compared with the medium Agar Candida ID (BioMérieux) and CHROMagar Candida (CHROMOagar Company Ltd.). The results of the comparison indicated that the Candida ID Agar could not differentiate strains of *Candida tropicalis* from those of *Candida kefyr*. It was also unable to differentiate *Candida guilliermondii* from the former two species. Chromogenic Agar Medium could only identify *Candida albicans*.

The new medium is faulty in the vitamins and microelements necessary for abundant growth of *Candida* species. Glucose is the only source of carbohydrates and energy in the medium; mycological peptone, by itself, does not ensure the other nutrients necessary for most of the species to be cultured. Likewise, the culture media CLED, nutritional Agar, Agar LAB Lemco, Agar for count in standard dishes and Agar yeast extract fail to give satisfactory results because of the abovementioned reasons.

An important disadvantage of all culture media studied in the abovementioned work, including the new medium, is the low stability of their ready-to-use form in Petri dishes, which lasted only 6 weeks as a maximum, primarily because of the labile nature of some of the substrates and reagents used. Mostly remarkable in this connection is Candida ID Agar, which must be incubated in the absence of light according to the manufacturer's instructions. Finally, it should be pointed out that CDA Agar, Candida ID Agar and Candida CHROMOagar were all incapable to efficiently differentiate *Candida* strains and/or species from each other. In particular, the species *Candida albicans* varied its color from green to purple blue and turquoise blue, leading to confusion with *Candida glabrata, Candida guilliermondii, Candida kefyr, Candida krusei, Candida lusitaneae, Candida parapsilosis, Candida pelliculosa, Candida rugosa* and *Candida tropicalis*. In the medium Candida ID Agar, some *Candida* species are liable to be confused, according to their coloration, with other yeast species.

The intents of incorporating the new chromogenic substrate to corn flour agar, potato and dextrose agar and rice extract agar did not facilitate identification or differentiation of *Candida* species. This conclusion confirms that none of the sources of vegetable origin traditionally employed in these media was able to provide the promoting metabolites intervening in the diverse metabolic cycles of the *Candida* species tested, and that none of the chromogenic or fluorogenic reactions hitherto used jointly with such nutrients have facilitated isolation, differentiation or identification of any of the *Candida* species of clinical or environmental relevance.

### DESCRIPTION OF THE INVENTION

The objective of this invention is to provide the composition of a culture medium intended for selective isolation, differentiation and simultaneous identification of fungus species, especially of the genus *Candida.*

The novelty of this invention lies on the inclusion, in the composition of the nutritional medium, of a mixture of sweet potato extract plus yeast extract or tomato extract in a quantity ranging from 20 to 30 g/L. Said mixture is composed of 50 to 67 % sweet potato extract plus 33 to 50 % yeast extract or 33 to 50 % tomato extract.

There exist no previous reports on the use of sweet potato extract in cultures of *Candida* species, which were surprisingly favored by the inclusion of this nutritional basis in the culture media.

Another novel aspect of this invention lies on combining the mixture of sweet potato extract plus yeast or tomato extract with other nitrogen sources, preferably glycine in a quantity ranging from 0 to 3 g/L, bacteriological peptone from 0 to 5 g/L and enzymatic casein hydrolysate from 0 to 5 g/L for a joint quantity of these sources ranging from 0 to 13 g/L in the composition of the nutritional medium, thereby resulting a new combination of nitrogen sources not previously reported in scientific literature.

The combination of the aforementioned nutrients with Gram-positive and Gram-negative microorganism inhibitors that are present in the nutritional medium differs from all what has been found in the state-of-the-art papers dealing with culture of *Candida* species. Said inhibitors are present in the prototype constituting the object of the present invention in quantities ranging from 0 to 10 g/L and are selected preferably among nalidixic acid in a quantity ranging from 0 to 0.05 g/L, rose bengal from 0 to 0.05 g/L, sodium deoxycholate from 0 to 0.5 g/L, sodium sulfite from 0 to 3 g/L, ammonium-bismuth citrate from 0 to 5 g/L, and tetraphenyltetrazolium chloride from 0 to 0.05 g/L.

This combination is also novel because the microbial growth promoters obtained from sweet potato had never before been combined with the aforementioned inhibitors, and because the inhibition had not ever before been achieved without the employment of labile substances which hinder the growth of *Candida.*

In this composition, for the first time in a culture medium, nalidixic acid has been added in a quantity ranging from 0 to 0.05 g/L in order to inhibit the growth of Gram-negative bacteria in the presence of *Candida.* This had not been done before because of the proven inhibiting effect of this antibiotic on this fungus species. This addition was possible, however, owing to its combination with a magnesium salt (magnesium sulfate), which unexpectedly suppressed the inhibiting effect of nalidixic acid on *Candida.*

Another original aspect of the present invention is the combination of substances propitiating the identification of diverse *Candida* species based on change of color and/or of fluorescence, apparition of halos and morphology of the colonies, and on the change of the characteristics of the medium with the nutrients that contributed by sweet potato extract.

These substances to be combined with the mixture of sweet potato extract and yeast extract are selected between the chromogenic and fluorogenic substrates to detect enzymatic activities.

A combination has been made, for the first time, of a group of chromogenic and/or fluorogenic substrates and proteolytic activity markers that is intended for simultaneous detection of enzymatic activities of the species of the genus *Candida* jointly with the phase of selective isolation from samples of clinical or environmental origin in the presence of activators for these reactions, which had not been previously reported for this purposes, such as trehalose or maltose in quantities ranging from 0 to 10 g/L.

For the first time, the detection of α-glucosidase activity has been used in the composition of an agar culture medium in order to differentiate or identify *Candida* species by chromogenic or fluorogenic reactions, especially *Candida albicans, Candida tropicalis* and *Candida parapsilosis*. For this purpose, methyl-umbellipheryl-α-D-glucopyranoside was incorporated in quantities up to 0.1 g/L. Likewise, the state-of-the-art papers show no description of inclusion in culture media for yeasts, especially for those of the genus *Candida,* of chromogenic and/or fluorogenic substrates intended to identify the phospholipase activity of species such as *Candida albicans, Candida tropicalis* and *Candida parapsilosis.* In the case of this invention, the ammonium salt of 5-bromo-4-chloro-3-indoxyl-mio-inositol-phosphate was included in quantities up to 0.1 g/L.

An unexpected effect resulted from combining a group of substrates to achieve, for the first time, differentiation of all *Candida* species of clinical relevance. Specifically, *Candida albicans* is identified by its activities: phospholipase (ammonium salt of 5-bromo-4-chloro-3-indoxyl-mio-inositol-phosphate), α-glucosidase (methyl-umbellipheryl-α-D-glucopyranoside), hexosaminidase (methyl-umbellipheryl-N-acetyl-β-D-galactopyranoside and 5-bromo-4-chloro-3-indoxyl-N-acetyl-β-D-glucosaminide) and L-proline aminopeptidase (L-proline-7-amido-4-methyl-coumarin hydrobromide and L-proline-para-nitroalinine); *Candida tropicalis* is identified by its activities: phospholipase (ammonium salt of 5-bromo-4-chloro-3-indoxyl-mio-inositol-phosphate) and α- and β-glucosidase (methyl-umbellipheryl-α-d-glucopyranoside, 5-bromo-4-chloro-3-indoxyl-β-d-glucopyranoside and 6-chloro-3-indoxyl-β-d-glucopyranoside); *Candida kefyr* is identified by its β-glucosidase activities (5-Bromo-4-chloro-3-indoxyl-β-D-glucopyranoside and 6-chloro-3-indoxyl-β-D-glucopyranoside) besides its proteolytic activity; *Candida parapsilosis* is identified by its activities: phospholipase (ammonium salt of 5-bromo-4-chloro-3-indoxyl-mio-inositol-phosphate), α-glucosidase (methyl-umbellipheryl-α-D-glucopyranoside) and L-proline aminopeptidase (L-proline-7-amido-4-methyl-coumarin hydrobromide and L-proline-paranitroaniline); *Candida krusei* and *Candida glabrata* are identified by their white color (opaque and brilliant, respectively) due to the absence, in these two species, of the aforementioned enzymatic activities.

Another novel element is the inclusion of a marker of proteolytic activity that is present only in *Candida kefyr*. In this case, surprisingly, casein of powdered skim milk in a quantity up to 10 g/L was hydrolyzed by this microorganism, whilst it was not by the remaining species.

The inclusion maltose or trehalose in the composition, with the presence of other carbon sources, surprisingly produced a marked increase in intensity of the chromogenic reaction (color) of the colonies. This effect is contradictory to the general commercial practice, which follows the employment of chromogenic and fluorogenic substrates in media which, in general, do not employ sugars or other carbon sources of a nature similar to that of the substrates in order to avoid a drastic fall in pH and, in consequence, a decrease in intensity of the chromogenic reaction, or to avoid the occurrence of a decrease in color intensity of the colonies due to other competition mechanisms, by means of which the microorganisms consume the most available form of sugar and metabolize the chromogenic substrate to a lesser extent.

Likewise original is the combination of the group of chromogenic and/or fluorogenic substances with other indicators or colorants, carbon sources and organic and inorganic salts existing in the nutritional medium in quantities ranging from 0 to 26 g/L, such as glycerol from 0 to 25 mUL, bromocresol purple from 0 to 0.04 g/L, bromothymol blue from 0 to 0.08 g/L and magnesium sulfate from 0 to 0.5 g/L.

The combination of the pH regulating substances in quantities ranging from 0 to 5 g/L, within which some of them act as enzymatic activators, such as phosphates, preferably monopotassium phosphate (from 0 to 1 g/L) and dipotassium phosphate (from 0 to 4 g/L), in an environment pH-adjusted with buffers, preferably 3-Morpholino-propansulfonsaure (3-morpholine-propane sulfonic acid), also known as MOPS (from 0 to 0.5 g/L), becomes singular because of the combination of their actions with those of the micro- and macroelements present in sweet potato extract, which surprisingly act as buffers and keep the pH value within a range appropriate to all *Candida* species.

Thus, surprisingly, an enhanced promotion of the growth of all *Candida* species was observed with the employment of the composition described in the present invention (113 % in average) with regard to the traditional media, even those formulated without any inhibitor, such as Sabouraud's Agar Dextrose, the medium par excellence to promote fungus growth.

This discovery was not evident from state-of-the-art papers, which establish that bacteria inhibitors, in the general case, inhibit to a certain extent the growth of the relevant microbial species. Thus, in general, when the recovery indexes of the selective media are calculated and compared with those of special media without inhibitors, the recounts and colonial morphologies are inferior with the use of said selective media.

The nutritional medium also includes agar in a quantity ranging from 13 to 15 g/L in order to ensure the development of the morphological characteristics that are typical of the colonies of the diverse *Candida* species, as well as the observation of chromogenic or biochemical reactions in the medium, such as the formation of transparent halos.

The original nutritional medium is composed also of distilled or deionized water, in which the other solid or liquid components are dissolved in quantities ranging from 35 to 50 g/L. The pH is adjusted to the range from 6.0 to 6.8.

For the first time, with the help of this nutritional medium, the *Candida* species of clinical or environmental relevance can simultaneously be selectively isolated, identified and differentiated in such a short time as 24 to 48 hours.

It is worthwhile to highlight that the nutritional medium with addition of the indicative substrates facilitates a new combination of morphological and biochemical approaches to identify *Candida* species.

This composition for isolation and differentiation of fungus species achieves, as main novelty and advantage, simultaneous isolation and identification of the greatest number of *Candida* species reported in the state-of-the-art papers; specifically, it is possible for the first time to differentiate 94 % of *Candida* species with clinical incidence, namely *Candida albicans, Candida tropicalis, Candida kefyr and Candida parapsilosis,* besides *Candida krusei.*

The advantages of the new nutritional medium are as follows:
- The nutritional medium ensures abundant growth of the diverse *Candida* species of clinical or environmental relevance, as it contains a wide range of nutrients contributed by sweet potato extract and its combination with the yeast extract or tomato extract. This abundant growth can be observed even after only 24 hours of incubation, without necessity of waiting for more than 48 or 76 hours for the colonies showing their peculiar morphological characteristics.
- The combination of sweet potato extract with yeast extract or tomato extract ensures suitable levels of macro-and microelements, vitamins, especially C and the B complex, as well as other water-soluble extractive substances enhancing the metabolic routes of the diverse *Candida* species and their specific enzymatic activity, which in turn promotes the occurrence of conventional chromogenic and fluorogenic reactions and of substrate decomposition reactions within a maximal period of 24 to 36 hours. This enhancement allows the inclusion of several biochemical markers in the formulation of the final medium in order to achieve the simultaneous identification of diverse *Candida* species in a single step.
- A remarkable advantage of the present invention lies on having achieved of a recovery of relevant *Candida* species higher than 110 % in the average, with regard to culture media without inhibitors specifically designed for fungus culture.
- The nutritional medium is simple in its preparation, needs no complicated additions, and the inoculation technique is the one commonly used.
- The identification of the diverse *Candida* strains and species is carried out visually by taking advantage of the diversity of colors of the colonies and of the culture medium, the formation of a transparent halo around the colony and, finally, the basic aspects of colony morphology (edges and surface). No specially trained or qualified personnel are needed for preparation of the culture medium or interpretation of the results.
- The contribution of nutrients from the mixture of sweet potato extract and yeast extract or tomato extract provides a nutritional medium that is capable to isolate, recover, differentiate and identify *Candida* colonies in a single step, without necessity of using a previous method or means for isolation of the colonies.
- The analytical sensitivity and accuracy of the culture medium are due to the fact that the identification does not depend on a group of biochemical indicators or markers, but only on enzymatic reactions that are typical of the diverse species, and on very well established morphological characteristics, without ambiguities or diversities between the diverse strains of any certain species.
- With the chromogenic components of the new composition and their combinations with skim milk, new forms of differentiation or identification of the relevant *Candida* species are achieved with more than one enzymatic marker, which in turn remarkably enhances the sensitivity and accuracy of diagnose with regard to previous solutions.
- The combination of sweet potato extract with yeast or tomato extract ensures that the new culture medium has significant contents of vitamins, especially A, B1, B5, B6, B12 and C supplied by the extracts, all of which are metabolites favoring exuberant growth of the diverse *Candida* species.
- The contribution of sweet potato extract and yeast or tomato extracts ensures, furthermore, contents of diverse carbohydrates that can replace all the requirements of the diverse species. For that reason, it is not necessary to include other carbohydrates to supply for the essential necessities of the species to be cultured. This fact, furthermore, facilitates species identification and differentiation based on the ability to decompose the substrates constituted by carbohydrates or containing groups that mimic said carbohydrates.
- The nutritional medium is favored by its contents in iron, calcium, phosphorus, magnesium, potassium, etc., among other microelements contributed by the sources from which the mixture of extracts is obtained, especially sweet potato. Therefore, it is not necessary to incorporate other salts as nutritional supplements for the *Candida* species to be isolated or identified. Nor is it necessary to provide a substantial additional contribution to activate the enzymatic reactions supporting the differentiation of *Candida* species by means of biochemical and/or chromogenic and fluorogenic tests. The addition of this type of substances to the new nutritional medium is only necessary in those cases where an additional complementation is required to obtain very quick responses from poor inocula or very low yeast concentrations in the samples.
- The chromogenic and/or fluorogenic reaction of identification of the diverse species is markedly higher in intensity with regard to the characteristics of other culture media described in the state-of-the-art papers, due to the enhancement effect attained by adding trehalose or maltose to the composition in previously indicated quantities.
- The combination of the mixture of sweet potato extract with yeast or tomato extract jointly with other nitrogen sources such as glycine, enzymatic casein hydrolysate or bacteriological peptone in previously stipulated ensure availability of different nitrogen forms (amino, amino acid, peptide, polypeptide and protein forms) which are able to supply the diverse requirements of the diverse species of cultured microorganisms in early stages of cellular development and, therefore, the colonies can reach full development in shorter time. On the other hand, that additional supply supports the conservation of the colonies in the Petri dishes for even longer periods of time.
- The nutritional medium does not contain substances which, because of its nature, could hinder biochemical, chromogenic or fluorogenic reactions, as are the cases of cycloheximide, chloramphenicol, gentamicin or rhodamine, either because they can interfere with the metabolic route of the specific enzyme-substrate action, or because they color the colony, thereby impeding the detection of color or fluorescence formation.
- The composition of the culture medium uses no thermolabile antibacterial substances inhibiting the growth of Gram-negative or Gram-positive microorganisms or other types of yeasts or molds, but combinations of more stable substances, such as nalidixic acid, sodium deoxycholate, sodium sulfite, ammonium citrate and/or rose bengal, which finally redounds in greater stability of the prepared nutritional medium, ready to be used in Petri dishes.
- On the other hand, the non necessity to include these inhibitors redounds in better recovery of the same *Candida* species the development of which is affected by such substances as tellurite, which, even at moderate concentrations, inhibit or slow down the growth.
- The addition of nalidixic acid favors a more effective inhibition of Gram-negative bacteria in comparison with previous solutions; furthermore, the composition becomes more stable to heat and in time.
- The use of nalidixic acid as inhibitor allowed, for the first time in a composition for identification of *Candida* species, the incorporation of enzymatic chromogenic or fluorogenic markers to detect phospholipase or α-glucosidase activities, since the other inhibitors commonly used provide for diverse chromatic reactions in the colonies of *Candida* species.
- It became possible to isolate *Candida* species selectively and directly from clinical or environmental samples owing to the inhibition of the accompanying bacterial flora, which can even be present at high concentrations (3-5 x 10⁸ UFC/MI), while *Candida* species show, in the average, 113 % recovery with regard to a general culture medium without inhibitors (Sabouraud's Dextrose Agar). This effect was achieved owing to the new combination of inhibitors to Gram-negative and Gram-positive microorganisms with the nutrients and magnesium salts.
- Likewise, the present invention does not use substances that are light-sensitive to such an extent that the incubation of the culture medium should have to be carried out in the absence of light.
- The combination of sweet potato extract with yeast or tomato extract completed with the diverse salts included in the formulation (K₂HPO₄, KH₂PO₄ MgSO₄) at concentrations indicated ensures a buffering capacity on a wide range of pH values (from 6.0 to 6.8) that is suitable to the diverse species of microorganisms to be detected and prevents, at the same time, growth inhibition caused by increased acidity resulting from accumulation of metabolites in the culture medium.
- The mixture of sweet potato extract and yeast extract allows the identification reactions, either biochemical, chromogenic or fluorogenic, to lapse in a natural way, activated by the micro- and microelements and vitamins, without need of using substances acting as cell permeability enhancers at high concentrations, such as bile salts, which cause inhibition of some *Candida* species.
- The incorporation of maltose or trehalose do not only enhance the chromogenic reaction in the colonies, but it supports also a more intense growth and the formation of colonies with size greater than that achieved with the culture media reported in the state-of-the-art papers.
- The presence of agar in quantity ranging from 13 to 15 g/L in the combination of the sum of the other solid components and the water added to prepare the nutritional medium ensures a firm support so that *Candida* colonies can develop their differential colors and morphological characteristics in such a combination that all the species tested can be isolated or recovered and simultaneously identified and/or differentiated from each other without employment of any complex inoculation technique or personnel specially trained.
- The concentrations of agar, other solid and liquid components and water support the detection of chromogenic or fluorogenic reactions, biochemical reactions, colonial or morphological characteristics and metabolite release or appearance of halos on the surface and/or in the depth of the culture medium; therefore, any reaction can be visualized not only on the top of the dish, but also on the bottom.
- The concentration of the nutritional medium in water varies from 35 to 50 g/L, with the concentration of sweet potato extract in the final nutritional medium always in the range from 10 to 20 g/L.

### DETAILED DESCRIPTION OF THE INVENTION

The detailed description of the invention consists on preparing a mixture of powdered sweet potato extract with yeast extract, with the sweet potato extract in a quantity from 50 to 67 % with regard to the mass of mixture. Likewise, the sweet potato extract can be mixed with tomato extract, with the sweet potato extract in a quantity from 50 to 67 % with regard to the mass of mixture. The mixture of sweet potato extract with yeast extract or tomato extract is added to the nutritional medium in a quantity ranging from 20 to 30 g/L.

The mixture can also be prepared by starting from the extracts in liquid form. In this case, the calculation for the mixture is carried out on the basis of the total solids contents in the two types of extracts to be mixed. If prepared by starting from any liquid ingredient, the quantity of mixture of liquid extracts will also be calculated by starting from the total solids contents in the mixture, and the rest of its mass will be subtracted from the volume of water to be used in preparing the nutritional medium.

The mixture is added to 1 L distilled or deionized water with pH in the range from 5 to 7 (the moisture content is previously subtracted, as pointed out before when mixing one or both extracts in liquid form).

The remaining substances contributing nitrogen are added in quantities ranging from 0 to 13 g/L. These are selected specifically among glycine at concentration from 0 to 3 g/L, bacteriological peptone from 0 to 5 g/L and enzymatic casein hydrolysate from 0 to 5 g/L.

Inhibitors are incorporated in a quantity ranging from 0 to 10 g/L. They are selected preferably among nalidixic acid in quantity from 0 to 0.05 g/L, rose bengal from 0 to 0.05 g/L, sodium deoxycholate from 0 to 0.5 g/L, sodium sulfite from 0 to 3 g/L, ammonium-bismuth citrate from 0 to 5 g/L, and tetraphenyltetrazolium chloride from 0 to 0.05 g/L.

Substances propitiating identification of the diverse *Candida* species are added also. They are selected among the chromogenic and fluorogenic substrates, indicators or colorants, which are present in the nutritional medium in a quantity ranging from 0 to 26 g/L, preferably the ammonium salt of 5-Bromo-4-chloro-3-indoxyl-mio-inositol-phosphate ranging from 0 to 0.1 g/L, methyl-umbellipheryl-α-D-glucopyranoside from 0 to 0.1 g/L, 5-bromo-4-chloro-3-indoxyl-β-D-glucosaminidine from 0 to 0.1 g/L, 5-bromo-4-chloro-3-indolyl-N-acetyl-β-D-glucopyranoside from 0 to 0.1 g/L, 4-methyl-umbellipheryl-N-acetyl-β-D-galactosaminide from 0 to 0.15 g/L, 6-chloro-3-indoxyl-β-D-glucopyranoside from 0 to 0.1 g/L, glycerol from 0 to 25 mUL, bromocresol purple from 0 to 0.04 g/L, bromothymol blue from 0 to 0.08 g/L, L-proline-7-amido-4-methyl-coumarin-hydrobromide from 0 to 0.1 g/L, L-proline para-nitroanilide from 0 to 0.2 g/L.

The compounds used to activate and enhance the chromogenic and fluorogenic reactions are added, among them glycerol from 0 to 25 mUL, maltose from 0 to 10 g/L, trehalose from 0 to 10 g/L and magnesium sulfate from 0 to 0.5 g/L.

Powdered skim milk is incorporated in concentration ranging from 0 to 10 g/L:

Furthermore, other pH regulating substances are added in quantities ranging from 0 to 5 g/L, preferably monopotassium phosphate in a quantity ranging from 0 to 1 g/L, dipotassium phosphate from 0 to 4 g/L and 3-Morpholino-propansulfonsaure (3-morpholine-propane sulfonic acid), also known as MOPS, from 0 to 0.5 g/L.

Agar is added in a quantity ranging from 13 to 15 g/L and the pH of the nutritional medium is adjusted to the range from 6.0 to 6.8. The culture medium prepared is melted up by using any of the well-known methods.

Next are shown some examples of application of the new nutritional medium based on the present invention.

### Example 1

Test to evaluate the nutritional capacity of the mixture of sweet potato extract with yeast or tomato extract within the formulation of the culture medium and the use of some chromogenic and/or fluorogenic substrates.

Two formulations of the nutritional medium (Media 1 and 2) were tested in comparison with a culture medium using malt extract (RI) as the source of nutrients.

**Table 1. Composition of the culture medium**

| Components | g/L | 1 | 2 | RI |
|---|---|---|---|---|
| Sweet potato extract | 10.0 | X | X | |
| Tomato extract | 10.0 | X | | |
| Yeast extract | 10.0 | | X | |
| Malt extract (reference) | 20.0 | | | X |
| Monopotassium phosphate | 1.0 | X | X | X |
| Magnesium sulfate | 0.5 | X | X | X |
| 5-bromo-4-chloro-3-indolyl-N-acetyl-β-D-glucopyranoside | 0.04 | X | X | X |
| 4-methyl-umbellipheryl-N-acetyl-β-D-galactosaminide | 0.04 | X | X | X |
| Agar | 15.0 | X | X | X |

The pH of the culture medium was adjusted to 6.5. After sterilization, the pH was stabilized to 6.0 in variant 1 and to 6.4 in variant 2. In the comparison medium RI the pH was kept at 6.3.

The formulation of Medium 2 is similar to that of Medium 1, but instead of the tomato extract, yeast extract was used in same concentration of 10 g/L.

The comparison medium (RI) is formulated with malt extract (Merck), at concentration of 20 g/L.

The microorganisms tested (isolated strains coming from the Faculty of Biology of the University of Havana) were the following: *Candida albicans* (2 strains), *Candida tropicalis, Candida glabrata, Candida parapsilosis, Candida kefyr* and *Candida krusei.*

The dishes were incubated at 30 °C for 24 and 48 hours. The colonies were visually examined and interpreted as follows:
- Blue colonies are typical of the species bearing the enzyme β-D-glucosidase, namely: *Candida tropicalis* and *Candida kefyr*.
- Colonies with blue fluorescence are indicative of species bearing the enzyme N-acetyl-β-D-galactosaminidase, namely: *Candida albicans* and strains of *Candida tropicalis*.
- White colonies correspond to strains lacking the enzymes β-D-glucosidase and N-acetyl-β-D-galactosaminidase. The species are identified according to morphological characteristics, such as: *Candida krusei, Candida glabrata* and *Candida parapsilosis.*

The results of the tests are shown in table 2. *Candida* species can be identified according to the cultural characteristics of the colonies.

After only 24 hours of incubation, surprisingly, the characteristics of growth of *Candida albicans* and *Candida tropicalis* were observed, and mainly the colonial characteristics in the culture media.

The colonies were of characteristic size, well developed, favored by the nutrients provided by sweet potato extract jointly with those coming in yeast extract or tomato extract.

Upon 48 hours, the exuberant growth of the colonies and their morphology were still remaining.

It was observed that the culture media 1 and 2, formulated with the mixture of sweet potato extract and yeast extract, facilitated the differentiation of *Candida* species by the combinations of color and fluorescence, while the use of malt extract (RI) as reference developed the characteristic blue color only in *Candida kefyr*.

With the combination of sweet potato extract and tomato extract, the concentration of the chromogenic substrate was insufficient. Hence, it was decided to increase the concentration in subsequent experiments.

It was proved likewise that the presence of carbohydrates in sweet potato extract and its mixture with yeast extract were not only beneficial in enhancing growth of the species tested, but they also did not interfere, and even favor the appearance of chromogenic and/or fluorogenic reactions from the substrates incorporated, thereby confirming full compatibility of the aforementioned extracts with the substrates.

Such was not the case with malt extract, which inhibited the appearance of fluorogenic reactions for *Candida tropicalis* upon 48 hours, and of chromogenic reactions for most of them, except for *Candida kefyr*.

Lastly, it was noted that the concentration of agar was appropriate. In fact, very well defined colonial characteristic were observed, the nutritional medium did not suffer scratching or ruptures on its surface, and the coloring reactions in the colonies were very clearly observed on the surface and down to the full depth of the culture medium. The fluorogenic reaction was also very clearly observed in the culture medium.

**Table 2. Identification of selected Candida species in tested culture media**

| Microorganism | Culture medium | Coloration of colony | | Fluorescence | Morphology |
|---|---|---|---|---|---|
| | | Blue | White | | |
| *C. albicans* | 1 | | X | X | - |
| | 2 | | X | X | - |
| | RI | | X | X | - |
| *C. tropicalis* | 1 | X (pale) | | X | - |
| | 2 | | X | X | - |
| | RI | | X | - | - |
| *C. glabrata* | 1 | | X | - | Bulging, shiny, regular edges |
| | 2 | | X | - | |
| | RI | | X | - | |
| *C. kefyr* | 1 | X (intense) | | - | - |
| | 2 | X (intense) | | - | - |
| | RI | X (intense) | - | - | - |
| *C. krusei* | 1 | | X | - | Flattened, opaque, irregular edges |
| | 2 | | X | - | |
| | | | X | - | |
| *C. parapsilosis* | 1 | | X | - | Rough surface, regular edges |
| | 2 | | X | - | |
| | RI | | X | - | |

### Example 2

The mixture of sweet potato extract and yeast extract was combined with chromogenic and fluorogenic substrates (5-bromo-4-chloro-3-indolyl-N-acetyl-β-D-glucopyranoside, 4-methyl-umbellipheryl-N-acetyl-β-D-galactosaminide; in higher concentrations, L-proline para-nitroanilide was added) and sodium deoxycholate was used as bacteriostatic agent (table 3).

The strains were groove inoculated until exhaustion of the inoculum was reached. The culture media were incubated at 30 °C for a period of 36 hours. Exuberant growth was achieved on both media, even upon 36 hours. The chromogenic and/or fluorogenic characteristics, as well as the morphological ones, supported differentiation of *Candida* species (table 4).

**Table 3. Composition of culture media 3 and 4**

| Components | g/L | 3 | 4 |
|---|---|---|---|
| Sweet potato extract | 10.0 | X | X |
| Yeast extract | 10.0 | X | X |
| Monopotassium phosphate | 1.0 | X | X |
| Magnesium sulfate | 0.5 | X | X |
| 5-bromo-4-chloro-3-indolyl-N-acetyl-β-D-glucopyranoside | 0.1 | X | |
| 4-methyl-umbellipheryl-N-acetyl-β-D-galactosaminide | 0.15 | X | |
| L-proline paranitroanilide | 0.05 | | X |
| Agar | 15.0 | X | X |
| Glycine | 1.0 | X | X |
| Sodium deoxycholate | 0.5 | X | X |

It was likewise established that the extracts composing the nutritional medium are compatible with the chromogenic and/or fluorogenic substrates, which can be visualized inside the cell, and those diffusing to the medium. The decomposition reactions of these substrates are favored by the presence of sweet potato extract and yeast extract.

**Table 4. Identification of Candida species tested in media 3 and 4**

| Microorganism | Culture medium | Coloration of colony | | | Fluorescence | Morphology |
|---|---|---|---|---|---|---|
| | | Blue | Yellow | White | | |
| *C. albicans* | 3 | | | X | X | |
| | 4 | | X | | | |
| *C. tropicalis* | 3 | X (pale) | | | | |
| | 4 | | | X | | |
| *C. glabrata* | 3 | | | X | | Bulging X, shiny, regular edges |
| | 4 | | | X | | |
| *C. kefyr* | 3 | X (intense) | | | | |
| | 4 | | | X | | |
| *C. parapsilosis* | 3 | | | X | | Rough surface, regular edges |
| | 4 | | X | | | |

### Example 3

The nutritional medium (Medium 5) was prepared in the laboratory the ingredients set out in table 5. It was distributed on Petri dishes and inoculated. Inoculation was carried out by dilutions until isolated colonies in the range from 30 to 300 UFC/dish were obtained.

Incubation was carried out at 30 °C for 36 to 48 hours.

The nutritional medium (table 6) was able to promote growth of all species tested. The compatibility of sweet potato extract or its mixture with yeast extract with higher concentrations of chromogenic and fluorogenic substrates was proved.

Unexpectedly, an abundant growth was observed to high dilutions (low concentration of the inoculum). This proved the high analytic sensitivity of the nutritional medium and its contribution with the nutrients necessary to the recovery of *Candida* species, mainly of sweet potato extract and its combination with yeast extract.

**Table 5. Formulation of the nutritional medium with higher concentrations of chromogenic and fluorogenic substrates**

| Components | g/L | Medium 5 |
|---|---|---|
| Sweet potato extract | 10.0 | X |
| Yeast extract | 10.0 | X |
| Monopotassium phosphate | 1.0 | X |
| Magnesium sulfate | 0.5 | X |
| 5-Bromo-4-chloro-3-indolyl-N-acetyl-β-D-glucoside | 0.1 | X |
| 4-Methyl-umbellipheryl-N-acetyl-β-D-galactosaminide | 0.15 | X |
| Agar | 15.0 | X |
| Sodium deoxycholate | 0.5 | X |

**Table 6. Results of cultures of Candida species in the nutritional medium at dilution 10⁻⁴**

| Microorganism | Medium | Coloration of colony | | Fluorescence | Morphology |
|---|---|---|---|---|---|
| | | Blue | White | | |
| *C. albicans* | 5 | | X | X | |
| *C. tropicalis* | 5 | X (pale, dark center) | | | |
| *C. g*/*abrata* | 5 | | X | | Bulging, shiny, regular edges |
| *C. kefyr* | 5 | X (intense) | | | |
| *C. parapsilosis* | 5 | | X | | Rough surface, regular edges |
| *C. krusei* | 5 | | X | | Flattened, opaque, irregular edges |

### Example 4

To evaluate the compatibility of sweet potato extract in its mixture with yeast extract and with the inhibiting substances of the Gram-positive microorganisms, the nutritional medium (6) was formulated with addition of sodium deoxycholate (table 7).

The formulation was not sterilized in autoclave. It was boiled and distributed on the dishes.

The inoculation was carried out by serial dilutions starting from a standardized inoculum.

**Table 7.**

| Components | g/L |
|---|---|
| Sweet potato extract | 10.0 |
| Yeast extract | 10.0 |
| Monopotassium phosphate | 1.0 |
| Magnesium sulfate | 0.5 |
| Agar | 15.0 |
| Sodium deoxycholate | 0.5 |

**Table 8. Growth of Candida species in the nutritional medium formulated with sodium deoxycholate**

| Microorganism | Color | Morphology |
|---|---|---|
| *C. albicans* | White | Smooth, bulging, round |
| *C. tropicalis* | White | Smooth, bulging, round |
| *C. glabrata* | White | Bulging, shiny, regular edges |
| *C. kefyr* | White | Half-flattened, round |
| *C. parapsilosis* | White | Rough surface, regular edges |
| *C. krusei* | White | Flattened, opaque, irregular edges |

The presence of deoxycholate facilitated the growth of tested yeasts (table 8), even at high dilutions (10⁻⁴), without any inhibition. This corroborates the growth-promoting ability of sweet potato extract in its combination with yeast extract, which favors the formation of typical colonies in the presence of a cell-wall permeability booster.

On the other hand, magnesium sulfate worked as an adjuvant of the definition of the morphological characteristic of diverse *Candida* strains, as a shinier, more intense white color with well defined edges was observed on the colonies.

### Example 5

In order to corroborate the growth promoting power of the culture medium in an early stage and to check the compatibility of sweet potato extract with other biochemical substrates that could be employed for differentiation of the diverse *Candida* species, glycerol and bromothymol blue were included (Media 7 and 8) (table 9).

**Table 9. Formulation of Media 7 and 8**

| Components | g/L | 7 | 8 |
|---|---|---|---|
| Sweet potato extract | 10.0 | X | X |
| Yeast extract | 10.0 | X | X |
| Monopotassium phosphate | 1.0 | X | X |
| Magnesium sulfate | 0.5 | X | X |
| Agar | 15.0 | X | X |
| Sodium desoxycholate | 0.5 | X | X |
| Glicerol | 10 (mL) | | X |
| Bromothymol blue | 0.08 | X | X |

Intense growth of *Candida albicans* and *Candida tropicalis* was observed after only 24 hours of incubation.

On the other hand, for the purpose of checking the stability of the identification reactions and the maintenance of the nutritional properties of the nutritional medium, a reading of the results was taken upon 76 hours of incubation.

The growth of the said species continued. Furthermore, the identification reactions kept stable during that incubation time (table 10).

**Table 10. Results of microbial growth upon 76 hours of incubation of culture media 7 and 8**

| Microorganism | Culture medium | Coloration of colony | | Morphology |
|---|---|---|---|---|
| | | Yellow | White | |
| *C. albicans* | 7 | | X | |
| | 8 | | X | |
| *C. tropicalis* | 7 | X | | |
| | 8 | X | | |
| *C. glabrata* | 7 | | X | Bulging, shiny, regular edges |
| | 8 | X | | |
| *C. kefyr* | 7 | | X | |
| | 8 | | X | |
| *C. krusei* | 7 | | X | Flattened, opaque, irregular edges |
| | 8 | | X | |
| *C. parapsilosis* | 7 | X | | Rough surface, regular edges |
| | 8 | X | | |

### Example 6

The use of evaluation of the employment of the chromogenic substrate 6-chloro-3-indoxyl-β-D-glucopyranoside was evaluated as a component of nutritional medium 9 for the differentiation of some *Candida* species (table 11)

**Table 11. Formulation of nutritional medium 9**

| Components | g/L |
|---|---|
| Sweet potato extract | 10.0 |
| Yeast extract | 10.0 |
| Monopotassium phosphate | 1.0 |
| Magnesium sulfate | 0.5 |
| Agar | 15.0 |
| Sodium deoxycholate | 0.5 |
| 6-chloro-3-indoxyl-β-D-glucopyranoside (Rose-glu) | 0.1 |

The culture medium was inoculated by surface incubation until reaching high dilutions (10⁻⁴-10⁻⁵).

The dishes were incubated for 48 hours. At that time, the colonies of *Candida tropicalis* and *Candida kefyr* had taken a pink coloration, which was stronger on *Candida kefyr*. It was proved that, even at dilutions higher than those previously tested, it was possible not only to recover *Candida* species, but also to obtain characteristic chromogenic responses, as pink coloration was observed on both species.

The high nutritional power of sweet potato extract in its mixture with yeast extract became evident, both as a nitrogen source and as a provider of vitamins and macro- and micro elements, as these two nutritional bases were the only nutrient sources in the formulation of the culture medium.

It was proved once again that, far from interfering with the chromogenic reactions, both extracts in combination favor the appearance of the chromogenic reactions at very low concentrations of the inoculum.

The high recovery power of the nutritional medium was established, since at dilution of 10⁻⁴ it was impossible to perform the recount in the dishes of the species *Candida glabrata* and *Candida krusei,* as they were countless, and only at the dilution 10⁻⁵ was it possible to carry out the recounts, which appear in table 12.

**Table 12. Growth of diverse Candida species in nutritional medium 9**

| Microorganism | UFC/dish at dilution | | Coloration of colony | | Morphology |
|---|---|---|---|---|---|
| | 10⁻⁴ | 10⁻⁵ | Pink | White | |
| *C. albicans* | 45 | 5 | | X | |
| *C. tropicalis* | 47 | 5 | X | | |
| *C. glabrata* | INC | 36 | | X | Bulging, shiny, regular edges |
| *C. kefyr* | 51 | 11 | X | | |
| *C. krusei* | INC | 50 | | X | Flattened, opaque, irregular edges |
| *C. parapsilosis* | 48 | 6 | | X | Rough surface, regular edges |

| | | | | | |
|---|---|---|---|---|---|
| INC - Countless. | | | | | |

### Example 7

The nutritional capacity of several components rich in nitrogen of protein origin (enzymatic casein hydrolysate and bacteriological peptone) with and without sodium deoxycholate was evaluated.

Experimental variants of the nutritional medium were tested (from 10 to 15) (table 13). Diverse nitrogen sources and sodium deoxycholate were added in comparison with the reference medium (R2): Sabouraud's Dextrose Agar (BioCen).

**Table 13. Composition of the experimental variants of the nutritional medium and of the reference medium**

| Components | g/L | 10 | 11 | 12 | R2 | 13 | 14 | 15 |
|---|---|---|---|---|---|---|---|---|
| Sweet potato extract | 10.0 | X | X | X | - | X | X | X |
| Yeast extract | 10.0 | X | X | X | - | X | X | X |
| Monopotassium phosphate | 1.0 | X | X | X | - | X | X | X |
| Magnesium sulfate | 0.5 | X | X | X | - | X | X | X |
| Enzymatic casein hydrolysate | 5.0 | - | X | - | X | - | X | - |
| Bacteriological peptone | 5.0 | - | - | X | X | - | - | X |
| Dextrose | 40.0 | - | - | - | X | - | - | - |
| Agar | 15.0 | X | X | X | X | X | X | X |
| Sodium deoxycholate | 0.5 | - | - | - | - | X | X | X |

Culture media 10, 11, 12 and R2 were sterilized in autoclave at 121 °C for 15 minutes. Culture media 13, 14 and 15 were melted and boiled for 2-3 minutes.

The pH of the experimental variants of culture media was 6.8 in all cases. The pH of medium R2 was 5.6.

Groove inoculation was carried out until exhaustion of the inoculum.

On observing the growth and the morphology of *Candida* colonies, the conclusion was drawn that, even though the addition of enzymatic casein hydrolysate and bacteriological peptone caused no significant growth enhancement, growth was not hindered anyway.

In comparison with medium R2, differences were not observed between medium R2 and nutritional medium variants for *C. albicans, C. parapsilosis* and *C. tropicalis*, even with the variants using sodium deoxycholate as inhibitor. It was proved that, even though the growth of *C. glabrata* and *C. krusei* gave smaller, less intensely colored colonies as a result of the addition of sodium deoxycholate and the absence of alternative carbohydrate sources, these species were detected and recovered without difficulty.

In comparison with medium R2, which contains no inhibitors and includes a high level of sugar (dextrose), the variants of the nutritional medium proved to be satisfactory. This unexpected effect leads to the conclusion that the mixture of sweet potato extract with yeast extract supplies the nutritional medium with enough nitrogen for the development of the diverse *Candida* species, even though the culture medium may be supplemented in order to provide nitrogen sources of protein origin with a view to preserve this microorganism during longer conservation periods without exhaustion of the nutrient sources coming from both extracts.

Another remarkable aspect is that all the variants of the nutritional medium kept on a stable pH of 6.8, which, together with the aforementioned nutritional elements, ensured appropriate growth of *Candida* species, even under conditions less favorable than those prevailing in the control variant (R2). This was of vital importance to achieve the characteristic morphological expression of the colonies and the good recovery achieved.

### Example 8

With a view to test the influence of the addition of sweet potato extract as a source of nutrients of all types (carbohydrates, nitrogen, vitamins and macro-and microelements) and of the addition of glycerol as a possible enzymatic marker for subsequent differentiation of *Candida* species, new variants of the nutritional medium were elaborated (16-19) (table 14) and compared with the reference medium (R2).

The diverse *Candida* species were groove inoculated until exhaustion of the inoculum was reached.

The pH of medium R2 was 5.6 and that of the experimental variants was of 6.8. The behavior of this indicator, already tested in previous example, was corroborated once again.

In all the variants tested, in such an early period as 24 hours, the growth of all yeast species tested was observed.

It was proven that the addition of glycerol favored the growth of all the species tested, since colonies with better development were obtained. For this reason, it becomes certain that glycerol does not affect microbial growth.

**Table 14. Composition of the nutritional medium**

| Components | g/L | 16 | R2 | 17 | 18 | 19 |
|---|---|---|---|---|---|---|
| Sweet potato extract | 10.0 | 20 (double concentration ) | - | X | X | X |
| Yeast extract | 10.0 | X | - | X | X | X |
| Monopotassium phosphate | 1.0 | X | - | - | X | X |
| Magnesium sulfate | 0.5 | X | - | - | X | X |
| Enzymatic casein hydrolysate | 5.0 | - | X | - | - | - |
| Bacteriological Peptone | 5.0 | - | X | - | - | - |
| Dextrose | 40.0 | - | X | - | - | - |
| Agar | 15.0 | X | X | X | X | X |
| Sodium deoxycholate | 0.5 | X | - | X | X | X |
| Glycerol | 10 mL | - | - | - | - | X |

An increment in the quantity of sweet potato extract is likewise favorable to the nutritional medium as, in spite of the presence of sodium deoxycholate, good growth of relevant species was achieved.

The absence of monopotassium phosphate and of magnesium sulfate in variant 17 did not lead to absence of growth, but did to a slight decrease in intensity of growth of C. glabrata and C. krusei in 24 hours. This proves that sweet potato in its combination with yeast extract is sufficient to supply the minimum essential requirements in macro- and microelements to promote growth of *Candida* species.

### Example 9

This experiment was carried out to test the usefulness of incorporating glycerol with a pH indicator to differentiate *Candida* species in the nutritional medium, also to compare that response with the addition of dextrose, the carbohydrate used in most of the solutions described in the state-of-the-art papers to promote growth of these yeasts.

Two chromogenic and fluorogenic substrates were incorporated in parallel in order to detect possible interference of the carbon sources added with the chromogenic and fluorogenic reactions.

The formulations of the nutritional medium (20-22) appear in table 15, where they are compared with the reference medium R3 prepared by starting from the formulation of R2 with addition of the chromogenic and fluorogenic substrates, a pH indicator and sodium deoxycholate. The pH of all the culture media was adjusted to 6.8.

The strains of the diverse *Candida* species and other microorganisms were groove inoculated on the surface of the culture medium. The medium was incubated for 24-48 hours.

Candida species and other microorganisms such as: *Enterobacter aerogenes, Salmonella typhimurium, Pseudomonas aeruginosa, Enterococcus faecalis* and *Staphylococcus aureus* were inoculated.
Table 16 shows the responses obtained with variants of the nutritional medium tested with diverse microorganisms.

**Table 15. Formulations of the experimental variants of the medium proposed and of the reference medium**

| Components | g/L | Culture media | | | |
|---|---|---|---|---|---|
| | | 20 | 21 | 22 | R3 |
| Sweet potato extract | 10.0 | X | X | X | - |
| Yeast extract | 10.0 | X | X | X | - |
| Monopotassium phosphate | 1.0 | X | X | X | - |
| Magnesium sulfate | 0.5 | X | X | X | - |
| Agar | 15.0 | X | X | X | X |
| Sodium deoxycholate | 0.5 | X | X | X | X |
| Glycerol | 10 mL | - | - | X | - |
| Enzymatic casein hydrolysate | 5.0 | - | - | - | X |
| Bacteriological Peptone | 5.0 | - | - | - | X |
| Dextrose | 20.0 | - | X | - | 40 (double concentration) |
| Bromothymol blue | 0.08 | X | X | X | X |
| 5-bromo-4-chloro-3-indolyl-N-acetyl-β-D-glucoside | 0.1 | X | X | X | X |
| 4-methyl-umbellipheryl-N-acetyl-β-D-galactosaminide | 0.15 | X | X | X | X |

**Table 16. Response of the microorganisms tested on diverse variants of the culture medium**

| Microorganism | Medium | Characteristics of colony | Characteristics of medium | Fluorescence | Morphology |
|---|---|---|---|---|---|
| *C. albicans* | 20 | White-yellow | Blue | Yes | 1 mm, smooth, round |
| | 21 | Yellow | Yellow | No | 1 mm, smooth, round |
| | 22 | White-yellow | Blue | Yes | 1 mm, smooth, round |
| | R3 | Yellow | Yellow | No | 1 mm, smooth, round |
| *C. tropicalis* | 20 | White grey to blue | Green | Slight | 2 mm, smooth, round |
| | 21 | White grey | Green | No | 2 mm, smooth, rotund |
| | 22 | White grey blue | to Green | Slight | 2 mm, smooth, round |
| | R3 | White grey | Green | No | 2 mm, smooth, round |
| *C. glabrata* | 20 | White | Blue | No | 0.5 mm, smooth, round |
| | 21 | Yellow-orange | Yellow | No | 1.5 mm, smooth, round |
| | 22 | Yellow | Yellow | No | 1 mm, smooth, round |
| | R3 | Yellow | Yellow | No | 1 mm, smooth, round |
| *C. kefyr* | 20 | Blue | Green | No | 0.5 mm, smooth, round |
| | 21 | Green | Yellow | No | 0.5 mm, smooth, round |
| | 22 | Blue | Green | No | 0.5 mm, smooth, round |
| | R3 | NC | NC | - | - |
| *C. parapsilosis* | 20 | White | Blue | No | 1 mm, irregular, round |
| | 21 | White-Yellow | Green | No | 1 mm, irregular, round |
| | 22 | White-yellow | Green | No | 1 mm irregular, round |
| | R3 | Yellow | Yellow | No | 1 mm, irregular, round |
| *C. krusei* | 20 | Transparent | Blue | No | 0.5 mm, flattened |
| | 21 | Yellow | Green | No | 1.5 mm, flattened |
| | 22 | White-yellow | Greenish yellow- | No | 1 mm, flattened |
| | R3 | Yellow | Yellow | No | 1.5 mm, flattened |
| *E. aerogenes* | 20 | Green-blue with intense green center | Blue | No | 2-4 mm, round |
| | 21 | Yellow-orange | Yellow | No | 1 mm, round |
| | 22 | Green | Green | No | 2-4 mm, round |
| | R3 | Yellow, transparent halo | Yellow | No | 2 mm, round |
| *S. typhimurium* | 20 | Intense green | Green-blue | No | 1 mm, round |
| | 21 | Yellow | Yellow | No | 1-2 mm, irregular edges, flattened |
| | 22 | Intense green | Green-blue | No | 1 mm, round |
| | R3 | Yellow | Yellow | No | 1 mm, round |
| *P. aeruginosa* | 20 | Transparent | Blue | No | Irregular, muciform |
| | 21 | Yellow | Yellow | No | Irregular, muciform |
| | 22 | Yellow | Green | No | Irregular, muciform |
| | R3 | Yellow | Yellow | No | Irregular, muciform |
| *E. faecalis* | 20 | NC | NC | - | - |
| | 21 | NC | NC | - | - |
| | 22 | NC | NC | - | - |
| | R3 | NC | NC | - | - |
| *S. aureus* | 20 | NC | NC | - | - |
| | 21 | NC | NC | - | - |
| | 22 | NC | NC | - | - |
| | R3 | NC | NC | - | - |

| | | | | | |
|---|---|---|---|---|---|
| NG- No growth. | | | | | |

With variants 21 and R3, where dextrose was included, the results of differentiation of the species tested was not satisfactory because the hydrolysis of this sugar carried out by several Candida species and other microorganisms inoculated gave the very same color and a high production of acid accompanied with pH depression, observation of the end point of the indicator and inhibition of chromogenic reactions due to pH depression. Such is the case of *Enterobacter, Salmonella* and *Pseudomonas* and of almost *Candida* species, except *Candida tropicalis, Candida krusei* and *Candida parapsilosis* in variant 21.

The inclusion of this sugar is not advisable.

The addition of glycerol to nutritional medium variant 22 in the quantity indicated, besides not hindering growth of relevant microorganisms in 24 hours, especially *Candida krusei* and *Candida glabrata*, supported, jointly with the chromogenic and fluorogenic substrates, appropriate differentiation diverse *Candida* species and even of other Gram-negative microorganisms which might grow and interfere, at least in theory, with the identification of relevant yeasts.

It was possible to differentiate *Candida albicans* specifically by the white color of colonies and the blue fluorescence; *Candida tropicalis* by the blue color of colony mass and blue fluorescence; *Candida kefyr* by the blue color without fluorescence; *Candida glabrata* by the yellow color of colonies; *Candida krusei* by yellowish white colonies, but its morphology is characterized by opaque, flattened colonies with irregular, but rounded edges; and *Candida parapsilosis,* of the same color, but with irregular surface.

The Gram-negative microorganisms stained the culture medium green and originated green colonies, except Pseudomonas aeruginosa, which produced yellow, but muciform colonies.

The presence of sodium deoxycholate supported inhibition of the Gram-positive microorganisms inoculated, even at high concentrations (10⁸ UFC/mL).

### Example 10

In order to assess the influence of pH on microbiological response, the pH was varied from 6.1 to 6.8 by adding diverse buffers (culture media 23-25) (table 17).

**Table 17. Formulation of culture media at pH value 6.1**

| Components | g/L | Culture media | | |
|---|---|---|---|---|
| | | 23 | 24 | 25 |
| Sweet potato extract | 10.0 | X | X | X |
| Yeast extract | 10.0 | X | X | X |
| Monopotassium phosphate | 1.0 | X | X | X |
| Magnesium sulfate | 0.5 | X | X | X |
| 5-bromo-4-chloro-3-indolyl-N-acetyl-β-D-glucoside | 0.1 | X | X | X |
| 4-methyl-umbellipheryl-N-acetyl-β-D-galactosaminide | 0.15 | X | X | X |
| Agar | 15.0 | X | X | X |
| Sodium deoxycholate | 0.5 | X | X | X |
| 3-(N-morpholine)-propane sulfonic acid (MOPS) | 0.5 | - | X | - |
| Dipotassium phosphate | 4.0 | - | - | X |

Culture media 26, 27 and 28 are based on same formulations as are the three media appearing in table 17, but with the pH adjusted to 6.8.

In general, it became evident that the nutritional medium variants with pH 6.1 took longer time to react in the presence of the substrate 5-bromo-4-chloro-3-indolyl-N-acetyl-β-D-glucoside for *Candida kefyr*. It should be highlighted, however, that *Candida albicans, Candida glabrata* and *Candida krusei* reached greater development at this pH that at pH 6.8.

The incorporation of MOPS did not exert negative influence on the development of the microorganisms tested, nor did it interfere with the identification response.

Also, the addition of dipotassium phosphate did not inhibit the response, but it caused the appearance of a slight opalescence that did not interfere with microbial identification or differentiation.

By way of conclusion of the experiment, it became evident that both pH values of 6.1 and 6.8 facilitated growth and differentiation of *Candida* species; nevertheless, the pH should be adjusted depending on the relevant species to be cultured or identified.

### Example 11

Chloramphenicol was included in the formulation of a variant of culture medium in order to check the effect sodium deoxycholate addition on *Candida* growth and identification in comparison with the employment, reported in the state-of-the-art papers, of antibiotic substances inhibiting growth of Gram-negative organisms.

Culture media 26 and 27 appearing in table 18 were compared with the chromogenic medium CHROMagar Candida (R4) (CHROMagar) and with Sabouraud's Agar Dextrose medium (R2) (Biocen).

Several strains of diverse *Candida* species were groove inoculated until reaching inoculum exhaustion.

Four isolated strains of *Candida* albicans, two of ATCC (10231 and 17111) and one strain of the remaining yeast species were inoculated. The results are shown in table 19.

**Table 18. Formulations of the nutritional medium with diverse inhibitors**

| Components | g/L | 26 | 27 |
|---|---|---|---|
| Sweet potato extract | 10.0 | X | X |
| Yeast extract | 10.0 | X | X |
| Monopotassium phosphate | 1.0 | X | X |
| Magnesium sulfate | 0.5 | X | X |
| Agar | 15.0 | X | X |
| Sodium deoxycholate | 0.5 | X | - |
| Chloramphenicol | 0.5 | - | X |
| 5-bromo-4-chloro-3-indolyl-N-acetyl-β-D-glucoside | 0.1 | X | X |
| 4-methyl-umbellipheryl-N-acetyl-β-D-galactosaminide | 0.15 | X | X |

**Table 19. Growth of Candida species in the nutritional medium and in CHROMagar Candida**

| Microorganism | Culture medium | Color of the colony | Fluorescence | Morphology | Size of colonies, mm/growth intensity |
|---|---|---|---|---|---|
| *C. albicans* | 26 | White | Blue | Smooth, round, bulging | 1-1.5 good |
| | 27 | White | Blue | Smooth, round, bulging | 1-1.5 good |
| | R2 | White | - | Smooth, round, bulging | 1 good |
| | R4 | Light green | - | Smooth, round, bulging | 1 good |
| *C. tropicalis* | 26 | Pale blue | Very weak | Smooth, round, bulging | 1.5-2 good-excellent |
| | 27 | White with slight blue tonality | Very weak | Smooth, round, bulging | 1.5-2 good-excellent |
| | R2 | White | - | Smooth, round, bulging | 1.5 good-excellent |
| | R4 | Blue-grey | - | Smooth, round, bulging | 1 good |
| *C. glabrata* | 26 | White | No | Smooth, round | 0.5 good |
| | 27 | White | No | Smooth, round | 0.5 good |
| | R2 | White | - | Smooth, round | 1-1.5 good |
| | R4 | Violet | - | Smooth, round | 1 good |
| *C. kefyr* | 26 | Intense blue | No | Smooth, round | 1 good |
| | 27 | Intense blue | No | Smooth, round | 1 good |
| | R2 | White | - | Smooth, round | 1 good |
| | R4 | White | - | Smooth, round | 1 good |
| *C. krusei* | 26 | White, semi-transparent | No | Opaque, flattened, cottony aspect | 0.5 good |
| | 27 | White, semi-transparent | No | Opaque, flattened, cottony aspect | good |
| | R2 | White, semi-transparent | - | Opaque, flattened, cottony aspect | 0.5 good |
| | R4 | Pale pink | - | Opaque, flattened, cottony aspect | 1 good |

As it may be seen, the incorporation of chloramphenicol interferes and hinders species differentiation, especially of the chromogenic reactions, for example, in the case of *Candida tropicalis*.

On the other hand, the use of chloramphenicol does not ensure persistent stability of the ready-to-use medium, mainly at room temperature.

The medium CHROMagar Candida was not used (conserved and incubated) in the presence of light because the manufacturer does not recommend that. Furthermore, the prepared medium could be used only for one day, because after this period it deteriorates.

The nutritional medium of the present invention prepared according to the formulation of variant 26 could be conserved even at ambient temperature without any deterioration of its growth-promoting characteristic or of the detection reactions.

With this formulation variant, it was possible to obtain colonies of *Candida albicans* and *Candida tropicalis* (the two species of greater importance) with sizes greater than it was by using the reference media.

### Example 12

The following experiment tested the influence of glycerol content in the presence of two pH indicators.

Table 20 shows the formulations of the first five variants (28-32). With the next five variants (33-37), the ingredients were kept at the concentration indicated, except for bromocresol purple, which was replaced by bromothymol blue.

All the formulations were sterilized in autoclave and the pH was adjusted to 6.8.

All strains were inoculated by surface grooving.

**Table 20. Basic ingredients of experimental medium in diverse compositions**

| Components | g/L | 28 | 29 | 30 | 31 | 32 |
|---|---|---|---|---|---|---|
| Sweet potato extract | 10.0 | X | X | X | X | X |
| Yeast extract | 10.0 | X | X | X | X | X |
| Monopotassium phosphate | 1.0 | X | X | X | X | X |
| Magnesium sulfate | 0.5 | X | X | X | X | X |
| Bromocresol purple | 0.04 | X | X | X | X | X |
| Agar | 13.0 | X | X | X | X | X |
| Sodium deoxycholate | 0.5 | X | X | X | X | X |
| Glycerol | Variable (mL) | - | 1 | 5 | 10 | 12.5 |

For *Candida albicans*, no growth-depressing effect was detected when glycerol concentration was increased. This microorganism does not use glycerol as substrate.

The medium did not change color; accordingly, no indicative enzymatic activity was detected, which otherwise would have manifested itself by a change in the pH of the medium.

Also with *Candida tropicalis* and *Candida parapsilosis,* no growth inhibition was observed when glycerol concentration was increased.

The resulting growth of *Candida kefyr* established that an increase in glycerol concentration is favorable to color, size and shine of the colonies. Likewise, this microorganism does not degrade glycerol as substrate.

An increment of glycerol facilitates better visualization of color change in the colonies of *Candida glabrata* (increase in yellow tonality) and of the culture medium (yellow); furthermore, the morphological characteristic are improved, as the colonies become more bulging, larger and more intensely colored. With this microorganism, bromocresol purple improved the response to the pH change. The color change became also visible on the bottom of the dish, as the reaction extended deeply, forming a yellow halo around the colony.

In the case of *Candida krusei,* an increase in glycerol content favored improved visualization of color change of the culture medium (yellow). Furthermore, the morphological characteristic improved with formation of larger colonies 1 to 3 mm in size and more accentuated white coloration. With this microorganism, bromothymol blue favored the detection of the color change in the culture medium (a turning to yellow) with increased substrate concentration. In general, the addition of agar in concentration of 13 g/L favored increased size of the colonies, albeit it is considered that, under this level, the surface of the culture medium can break during groove inoculation.

### Example 13

Several inhibitors to Gram-negative and Gram-positive microorganisms were tested (table 21) with a view to determine which of them should be used in the composition of the culture media of the present invention and to compare their results with those of other inhibitors previously added and reported by diverse authors and appearing in the state-of-the-art papers.

Six *Candida* species and six strains of Gram-positive and Gram-negative microorganisms were inoculated (by surface grooving with an inoculum 10⁸ UFC/MI) for inhibition, including *Proteus vulgaris, Salmonella typhimurium, Pseudomonas aeruginosa, Escherichia coli, Enterococcus faecalis* and *Staphylococcus aureus.*

The dishes with the culture media were incubated at 30 °C for 48 hours. Culture media variants 38 and 39 supported total inhibition of the Gram-negative and Gram-positive microorganisms tested. *Candida* species showed good growth. The color of the diverse species was dark brown, except for *Candida glabrata*, colored in white.

Variant 40 inhibited all Gram-positive microorganisms and *Proteus vulgaris*. All strains of *Candida* showed very abundant growth, with their characteristic morphology and pink color.

Nutritional medium 41 inhibited *Candida krusei* and *Candida parapsilosis,* but total inhibition of Gram-negative microorganisms was not achieved.

Variants 42 and 43 resulted too much inhibiting to the microorganisms to be inhibited, even to *Candida,* which could not grow in the medium.

Likewise, variants 44 to 48 inhibited *Candida* species totally.

The results of the tests unexpectedly proved that only sodium sulfite, ammonium-bismuth citrate and rose bengal, in combination with sweet potato extract and its mixture with yeast extract supported growth and differentiation of *Candida* species and were able to inhibit competing microorganisms, preventing Gram-negative organisms having the same identification reactions to be completely inhibited, the same as Gram-positive ones prevented to grow in the culture medium variants containing such ingredients.

**Table 21. Formulations of culture media with diverse inhibitors**

| Components | g/L | 38 | 39 | 40 | 41 | 42 | 43 | 44 | 45 | 46 | 47 | 48 |
|---|---|---|---|---|---|---|---|---|---|---|---|---|
| Sweet potato extract | 10.0 | X | X | X | X | X | X | X | X | X | X | X |
| Yeast extract | 10.0 | X | X | X | X | X | X | X | X | X | X | X |
| Monopotassium phosphate | 1.0 | X | X | X | X | X | X | X | X | X | X | X |
| Magnesium sulfate | 0.5 | X | X | X | X | X | X | X | X | X | X | X |
| Agar | 15.0 | X | X | X | X | X | X | X | X | X | X | X |
| Sodium deoxycholate | 0.5 | | | | | | X | | | | | |
| Sodium sulfite | 3.0 | X | X | | | | | | | | | |
| Ammonium-bismuth citrate | 5.0 | X | X | | | | | | | | | |
| Glycine | 3.0 | | X | | | | | | | | | |
| Rose bengal | 0.05 | | | X | | | | | | | | |
| Potassium tellurite solution 2 % | 18 mL | | | | X | X | X | | | | | |
| Lithium chloride | 5.0 | | | | | X | | | | | | |
| Cetrimide | variable | | | | | | | 0.3 | 1.0 | | | |
| Boric acid | variable | | | | | | | | | 1.0 | 3.0 | 5.0 |

### Example 14

An experiment was performed in order to test the effect of skim milk as a nutrient as regards its contribution with proteins and sugars (lactose).

Table 22 shows the composition of an experimental design intended to compare diverse nutritional compositions with two concentration levels of powdered skim milk. The responses were compared with a basic composition of the present invention without inclusion of milk (variant 49 of table 22) and with two formulations (variant 49 and variant 50) which are not claimed by the solution herein proposed, as they include sweet potato extract alone.

**Table 22. Ingredients of the experimental medium**

| Components | g/L | 49 | 50 | 51 | 52 |
|---|---|---|---|---|---|
| Sweet potato extract | 10.0 | X | X | | |
| Mixture of sweet potato extract and yeast extract | 20.0 | | | X | X |
| Skim milk | variable | 5 | 10 | | 5 |
| Monopotassium phosphate | 1.0 | X | X | X | X |
| Magnesium sulfate | 0.5 | X | X | X | X |
| Sodium deoxycholate | 0.5 | X | X | X | X |
| Glycerol | 10 (mL) | X | X | X | X |
| Agar | 15.0 | X | X | X | X |

Working pH was defined at 6.8.

All the variants of the composition tested were heated to boiling during 2 or 3 min after reaching the melting point of the agar. Autoclave sterilization was not applied to these variants.

Several species of the genus *Candida* were inoculated, such as *Candida albicans, Candida tropicalis, Candida krusei, Candida kefyr, Candida parapsilosis, Candida glabrata* and *Saccharomyces cerevisiae*.

The dishes were incubated during 48 hours at temperature of 25 °C. According to the readings, variants 49 and 50 did not achieved satisfactory development of the species *Candida glabrata, Candida krusei* and *Candida parapsilosis.* This can be due to shortage of such essential elements as vitamins and growth factors which are present in yeast extract.

Variants 51 and 52 showed a satisfactory growth for all microorganisms tested; however, it should be highlighted that in variant 49 *Candida kefyr* colonies unexpectedly showed a transparent halo due to the proteolytic effect on casein. No other species showed any proteolytic activity. Thus, the conclusion may be drawn that powdered skim milk can be used as a new marker of proteolytic activity for *Candida kefyr* to identify it or to differentiate it from the remaining species.

### Example 15

The antibacterial effect of nalidixic acid was tested in combination with sodium deoxycholate against a group of Gram-negative and Gram-positive bacteria and yeasts of the genus *Candida.*

The experiment was based on a design including a scale of concentrations of nalidixic acid from 0.01 to 0.05 g. Table 23 shows the compositions assessing the inhibition ability on the bacteria and the promotion of the growth of the fungus species.

The culture media were not sterilized in autoclave, they only were melted until reaching complete dissolution of the agar during 2 to 3 min. They were distributed in sterile dishes.

Final pH of culture media prepared was 6.8.

As seen in Table 23, the microbial species cultured were as follows: *Escherichia coli, Enterobacter aerogenes, Shigella flexneri, Salmonella typhimurium, Proteus vulgaris, Pseudomonas aeruginosa, Candida albicans, Candida tropicalis, Candida kefyr, Isachenkia orientalis (Candida krusei), Candida glabrata* and *Candida parapsilosis*.

The incubation was carried out starting from a standardized inoculum at 50 % transmittance by the method of exhaustion of the inoculum on the surface of the agarized culture medium in order to obtain isolated colonies.

The plates were incubated during 48 hours at temperature of 28 ± 2°C. According to test results, nalidixic acid at all three concentrations tested inhibited all Gram-negative bacteria, except for *Pseudomonas aeruginosa*. As for the yeasts of the genus *Candida,* it was surprisingly seen that, instead of causing growth inhibition, nalidixic acid favored colonial development of such species as *Candida krusei* and *Candida glabrata*, whereas other species of this genus were also favored, but less evidently.

**Table 23. Compositions designed to test the effects of nalidixic acid and sodium deoxycholate as bacteria inhibitors**

| Components | g/L | 53 | 54 | 55 |
|---|---|---|---|---|
| Mixture of sweet potato extract and yeast extract | 20.0 | X | X | X |
| Monopotassium phosphate | 1.0 | X | X | X |
| Magnesium sulfate | 0.5 | X | X | X |
| Sodium deoxycholate | 0.5 | X | X | X |
| Glycerol | 10 (mL) | X | X | X |
| Nalidixic acid | Variable | 0.01 g | 0.03 g | 0.05 g |
| Agar | 15.0 | X | X | X |

This effect was unexpected. Deepening in the possible causes, The conclusion can be drawn that magnesium ions in the quantity added (0.5 g/L as magnesium sulfate) increase the resistance of *Candida* to nalidixic acid.

The discovery of no inhibition of *Pseudomonas aeruginosa* is a matter of interest because this microorganism is far from standing as an unwanted competitor in *Candida* diagnosis, and its joint identification with *Candida* is interesting. For example, otomycosis has been considered by many authors as of fungous etiology while, in fact, it is the bacteria which are responsible for most cases of external otitis, whereas the fungi are only implied in a scarce number of such cases. It is considered that external otitis lies behind 5 to 20 % of otology consultations, most of them of bacterial etiology. External otitis is caused primarily by *Pseudomonas*, and only 15 to 20 % of these are attributed to fungi.

### Example 16

A group of experimental variants were tested with two levels of nalidixic acid in combination with sodium deoxycholate as Gram-negative and Gram-positive bacteria inhibitors, as was their combination with glycerol and Tween 80 with a view to define their effect in the development of diverse fungus species.

Table 24 shows the compositions used in this experimental design.

**Table 24. Compositions to test two levels of nalidixic acid in combination with glycerol and Tween 80**

| Components | g/L | 56 | 57 | 58 | 59 | 60 | 61 | 62 | 63 | 64 |
|---|---|---|---|---|---|---|---|---|---|---|
| Mixture of sweet potato extract and yeast extract | 20.0 | X | X | X | X | X | X | X | X | X |
| Monopotassium phosphate | 1.0 | X | X | X | X | X | X | X | X | X |
| Magnesium sulfate | 0.5 | X | X | X | X | X | X | X | X | X |
| Sodium deoxycholate | 0.5 | X | X | X | X | X | X | X | X | X |
| Nalidixic acid | Variable | 0.03 | 0.05 | - | 0.0 3 | 0.05 | - | 0.03 | 0.05 | - |
| Agar | 15.0 | X | X | X | X | X | X | X | X | X |
| Glycerol | 10 (mL) | - | - | - | X | X | X | - | - | - |
| ween 80 | 10 (mL) | - | - | - | - | - | - | X | X | X |

The pH was adjusted to 6.8. These variants were heated to boiling in order to attain complete homogenization of the product. Autoclave sterilization was not used.

The compositions were tested by the grooving method until exhaustion of the inoculum of the species: *Candida albicans, Candida tropicalis, Candida kefyr, Candida glabrata, Isachenkia orientalis (Candida krusei)* and *Candida parapsilosis.*

The dishes were incubated during 48 hours at temperature of 28 ± 2 °C.

The results show that the effect of glycerol (variants 59, 60 and 61) as a supplement in the culture medium gave rise to greater colonial development for all species tested than that resulting from using Tween 80 as a supplement. These results were evident after only 24 hours.

The variants with nalidixic acid at both concentrations (0.03 and 0.05 g/L), when combined with glycerol, showed that growth and development of the colonies of *Candida* species was not suppressed, but favored instead. With Tween 80 the result was less dramatic.

### Example 17

Several chromogenic and fluorogenic substrates were tested with diverse chromogenous and/or fluorogenous groups for glucosidase enzymes (hexosaminidase and β-glucosidase) present in certain species of the genus

*Candida*. The experimental design followed is shown in table 25.

**Table 25.**

| Components | g/L | 65 | 66 | 67 | 68 |
|---|---|---|---|---|---|
| Mixture of sweet potato extract and yeast extract | 20.0 | X | X | X | X |
| Monopotassium phosphate | 1.0 | X | X | X | X |
| Magnesium sulfate | 0.5 | X | X | X | X |
| Sodium deoxycholate | 0.5 | X | X | X | X |
| Glycerol | 10 (mL) | X | X | X | X |
| Agar | 15.0 | X | X | X | X |
| 4-MU-β-D-galactosaminide | 0.1 | X | - | - | - |
| X-β-D-galactosaminide | 0.1 | - | X | - | - |
| X-β-D-glucosaminide | 0.1 | - | - | X | - |
| X-β-D-glc | 0.1 | X | - | - | - |
| Salmon^{™}-β-D-glc | 0.1 | - | X | - | - |

| | | | | | |
|---|---|---|---|---|---|
| Legend: 4-MU-β-D-galactosaminide 4-methyl-umbellipheryl-N-acetyl-β-D-galactosaminide X-β-D-galactosaminide 5-bromo-4-chloro-3-indoxyl-N-acetyl-β-D-galactosaminide X-β-D-glucosaminide 5-bromo-4-chloro-3-indoxyl-N-acetyl-β-D-glucosaminide X-β-D-glc 5-bromo-4-chloro-3-indoxyl-N-acetyl-β-D-glucopyranoside Salmon^{™}-β-D-glc 6-chloro-3-indoxyl-N-acetyl-β-D-glucopyranoside | | | | | |

The compositions were adjusted to pH 6.8 and melted during 2-3 minutes until complete dissolution of the agar. They were not sterilized in autoclave.

The species inoculated were *Candida albicans, Candida tropicalis, Candida kefyr, Candida glabrata, Isachenkia orientalis (Candida krusei)* and *Candida parapsilosis*.

The inoculation of the species was carried by the method of serial dilutions, for the purpose of detecting the morphological and cultural characteristic of isolated colonies on diverse substrates.

The dishes were incubated at 30°C during 48 hours.

Table 26 shows the favorable influence of the presence of glycerol as a nutrient favoring development of the *Candida* species tested.

**Table 26. Growth of diverse Candida species in chromogenic and/or fluorogenic compositions**

| Microorganism | Variant | UFC/dish in dilution 10⁻⁵ | Characteristics of colonies | | | | |
|---|---|---|---|---|---|---|---|
| | | | Color | | | Fluorescence | Morphology |
| | | | Blue | Pink | White | | |
| C. albicans | 65 | 4 | - | - | X | X | Round, smooth, bulging, shiny, ∅ ≈ 1 mm |
| | 66 | 10 | - | - | X | - | |
| | 67 | 3 | X | - | - | - | |
| | 68 | 8 | - | - | X | - | |
| C. tropicalis | 65 | 9 | X | - | - | - | Round, smooth, bulging, shiny, ∅ ≈ 1.2mm |
| | 66 | 4 | - | X | - | - | |
| | 67 | 2 | - | - | X | - | |
| | 68 | 4 | - | - | X | - | |
| C. glabrata | 65 | 7 | - | - | X | - | Round, smooth, bulging, shiny, ∅ ≤ 1 mm |
| | 66 | 9 | - | - | X | - | |
| | 67 | 3 | - | - | X | - | |
| | 68 | 9 | - | - | X | - | |
| C. kefyr | 65 | 3 | X | - | - | - | Round, smooth, bulging, shiny, ∅ ≈ 1 mm |
| | 66 | 5 | - | X | - | - | |
| | 67 | 1 | - | - | X | - | |
| | 68 | 6 | - | - | X | - | |
| C. krusei | 65 | 8 | - | - | X | - | Flattened, opaque, irregular edges, ∅ ≈ 2-4 mm variable |
| | 66 | 6 | - | - | X | - | |
| | 67 | 6 | - | - | X | - | |
| | 68 | 8 | - | - | X | - | |
| C. parapsilosis | 65 | 11 | - | - | X | - | Rough surface, regular edges |
| | 66 | 13 | - | - | X | - | |
| | 67 | 10 | - | - | X | - | |
| | 68 | 9 | - | - | X | - | |

There appeared evident chromatic and fluorescent characteristics in all the variants tested. Particularly outstanding was the ability of *Candida albicans* to use galactosaminide and glucosaminide substrates, and that of *Candida tropicalis* to use chromogenic substrates of the type glucopyranoside.

The action of the enzyme galactosaminidase, present in *Candida albicans*, is seen more clearly in variant 65, where the colonies are identified by their fluorescence when illuminated with an UV lamp. However, the use of the chromogenic substrate to detect that very same enzymatic activity (variant 66) did not yield positive results, as the colonies did not take coloration. This unexpected, surprising negative response is characteristic of the specific composition claimed by this invention.

In variant 67 the isolated colonies of *Candida albicans* acquired a blue coloration due to the employment of the chromogenic substrate 5-bromo-4-chloro-3-indoxyl-N-acetyl-β-D-glucosaminide.

The colonies of *Candida tropicalis* and *Candida kefyr* took up a blue and pink coloration for variants 65 and 66, respectively, depending on the incorporation of component X or Pink to the glycoside, latter of which is used by both species.

Other species tested, such as *Candida parapsilosis, Candida krusei* and *Candida glabrata* do not respond to any of the substrates incorporated; therefore, they must be identified by their morphological characteristics.

### Example 18

A test was applied to a composition involving the use of broad-spectrum antibacterial agents and specific chromogenic substrates for identification and differentiation of *Candida species*.

The test was carried out with the formula appearing in table 27. Chromagar Candida (Chromagar, France) was used as the reference medium. The composition was prepared by weighing all ingredients and using deionized water. The pH was adjusted to 6.8. The composition was heated to boiling until complete dissolution of the agar during 2 to 3 min.

The reference medium was prepared according to the manufacturer's instructions printed on the label, with final pH 5.9.

A group of fungus and bacteria species were tested with a view to assess the inhibition ability and growth promotion ability of the experimental composition, including: *Candida albicans* ATCC 102321, *Candida tropicalis, Candida kefyr, Candida krusei (Isachenkia orientalis), Candida glabrata (Torulopsis), Candida parapsilosis, Saccharomyces cerevisiae* ATCC 9763, *Saccharomyces uvarum* ATCC 9080, *Pseudomonas aeruginosa* ATCC 27853, *Proteus vulgaris* ATCC 13315, *Salmonella typhimurium* ATCC 14028, *Shigella flexneri* ATCC 12022, *Escherichia coli* ATCC 25922, *Klebsiella pneumoniae* ATCC 13883, *Enterococcus faecalis* ATCC 19433 and *Staphylococcus aureus* ATCC 25923.

The results were very satisfactory. All Gram-positive and Gram-negative bacteria inoculated into the culture medium were inhibited, except for *Pseudomonas aeruginosa.* Differentiation of the species *Candida albicans, Candida tropicalis* and *Candida kefyr* was possible according to the coloration of the isolated colonies. Furthermore, *Candida krusei* and *Candida parapsilosis,* could also be identified by their white coloration, one opaque, the other shiny, respectively, and by their colonial morphology, with the composition of the present invention.

The reference medium, Chromagar Candida, facilitates identification of the species *Candida albicans, Candida tropicalis* and *Candida krusei* only, and attains total inhibition of the bacteria tested.

**Table 27. Composition of the chromogenic medium according to the present invention**

| Components | g/L | 69 |
|---|---|---|
| Mixture of sweet potato extract and yeast extract | 20.0 | X |
| Monopotassium phosphate | 1.0 | X |
| Magnesium sulfate | 0.5 | X |
| Sodium deoxycholate | 0.5 | X |
| Agar | 15.0 | X |
| Glycerol | 10 mL | X |
| Nalidixic acid | 0.03 | X |
| X-β-D-glucosaminide | 0.1 | X |
| Salmon^{™}-β-D-glc | 0.1 | X |

| | | |
|---|---|---|
| Legend: X-β-D-glucosaminide: 5-Bromo-4-chloro-3-indoxyl-N-acetyl-β-D-glucosaminide Salmon^{™}-β-D-glc: 6-chloro-3-indoxyl-N-acetyl-β-D-glucopyranoside | | |

### Example 19

For detection of other enzymatic activities in the species of the genus *Candida,* an experimental design was implemented in order to define with which of the substrates tested it would be possible to identify the greatest number of relevant fungus species. This experimental design was indispensable, since it was not obvious (example 17) that, in present composition, different substrates for a same activity gave same responses; even negative and contradictory responses were obtained for some species.

The variants consisted on incorporating the following chromogenic and fluorogenic substrates appearing in table 28 in concentration of 0.1 g/L. The ingredients were incorporated to the basic composition corresponding to variant 69 in table 25, according to the design presented in table 28.

**Table 28. Assessment of other chromogenous substrates for detection of esterase, phospholipase and glucosidase activities**

| Formulation | g/L | 70 | 71 | 72 | 73 | 74 | 75 | 76 | 77 | 78 |
|---|---|---|---|---|---|---|---|---|---|---|
| Composition of variant 20 of table 4 | 37.3 | | | | | | | | | |
| Magenta caprylate | 0.1 | X | - | - | - | - | - | - | - | - |
| X-nonanoate | 0.1 | - | X | - | - | - | - | - | - | - |
| -X-phos-inositol | 0.1 | - | - | X | - | - | - | - | - | - |
| 4-MU-β-D-xyl | 0.1 | - | - | - | X | - | - | - | - | - |
| X-α-D-manoside | 0.1 | - | - | - | - | X | - | - | - | - |
| X-α-D-gal | 0.1 | - | - | - | - | - | X | - | - | - |
| X-β-D-glcUA Na | 0.1 | - | - | - | - | - | - | X | - | - |
| Salmon^{™}-β-D-glc | Var. | - | - | - | - | - | - | - | 0. 1 | 0. 5 |

| | | | | | | | | | | |
|---|---|---|---|---|---|---|---|---|---|---|
| Legend: Magenta caprylate: 5-bromo-6-chloro-3-indoxyl-caprylate X-nonanoate: 5-bromo-4-chloro-3-indoxyl nonanoate X-phos-inositol: 5-bromo-4-chloro-3-indoxyl-mio-inositol-1-phosphate, ammonium salt 4-MU-β-D-xyl: 4-methyl-umbellipheryl-β-D-xylopyranoside X-α-D-manoside: 5-bromo-4-chloro-3-indoxyl-α-mannopiranoside X-α-D-gal: 5-bromo-4-chloro-3-indoxyl-α-galactoside X-β-D-glcUA . Na: 5-bromo-4-chloro-3-indoxyl-β-D-glucuronic acid, cyclohexylammonium salt Salmon^{™}-β-D-glc: 6-chloro-3-indoxyl-N-acetyl-β-D-glucopyranoside | | | | | | | | | | |

The compositions were pH adjusted to 6.8 ± 0.1 and sterilized by boiling during 2-3 minutes until complete dissolution of the agar. They were then inoculated by grooving until exhaustion of the inoculum. The following species were used: four isolated strains of *Candida albicans, Candida albicans* ATCC 10231 and *Candida albicans* ATCC 17111, *Candida tropicalis, Candida kefyr, Candida krusei, Isachenkia orientalis, Candida glabrata, Candida parapsilosis Saccharomyces uvarum* ATCC 9080 and *Pseudomonas aeruginosa* ATCC 27853.

The dishes were incubated 30°C during 48 hours.

In general, it was possible to detect which chromogenic or fluorogenic substrates were decomposed by the diverse *Candida* species.

*Candida albicans* is identified by its phospholipase (ammonium salt of 5-bromo-4-chloro-3-indoxyl-mio-inositol-phosphate) and β glucosidase activities, but only at a very high concentration of the substrate (6-chloro-3-indoxyl-β-D-glucopyranoside).

*Candida tropicalis* is identified by its phospholipase (ammonium salt of 5-bromo-4-chloro-3-indoxyl-mio-inositol-phosphate), glucosidase (6-chloro-3-indoxyl-β-D-glucopyranoside) and mannosidase (5-bromo-4-chloro-3-indoxyl-α-D-mannopiranoside) activities.

*Candida kefyr* is identified by its β glucosidase (6-chloro-3-indoxyl-β-D-glucopyranoside) activity.

*Candida parapsilosis* is identified by its phospholipase (ammonium salt of 5-bromo-4-chloro-3-indoxyl-mio-inositol-phosphate) and xilosidase (methyl-umbellipheryl-β-D-xylopyranoside) activities.

*Candida krusei, Isachenkia orientalis* and *Candida glabrata* are identified by their white color (opaque or shiny, respectively) due to the absence of the aforementioned enzymatic activities.

*Saccharomyces uvarum* ATCC 9080 is identified by its phospholipase activity. *Pseudomonas aeruginosa* ATCC 27853 did not respond to none of the substrates used, although the colonies showed greenish fluorescence when impacted by UV light.

### Example 20

The composition described in variant 68 of table 25 was mixed with the chromogenic and fluorogenic substrates described in table 29.

Each variant was tested at 3 different pH values: 6.0, 6.4 and 6.8.

The following results were obtained:

*Candida albicans* is identified by its L-proline aminopeptidase (L-proline-7-amido-4-methyl-coumarin hydrobromide) and α-glucosidase (methyl-umbellipheryl-α-D-glucopyranoside) activities; *Candida tropicalis* and *Candida parapsilosis* are identified by their α-glucosidase (methyl-umbellipheryl-α-D-glucopyranoside) activity. *Candida parapsilosis* additionally responded to the substrate for L-proline aminopeptidase (L-proline-7-amido-4-methyl-coumarin hydrobromide).

**Table 29. Evaluation of other chromogenic substrates for detection of aminopeptidase, glucosidase and alkaline phosphatase activities**

| Formulation | g/L | 79 | 80 | 81 | 82 | 83 | 84 |
|---|---|---|---|---|---|---|---|
| Composition of variant 20 in table 4 | 37.3 | X | X | X | X | X | X |
| H-Pro-AMC-HBr | 0.1 | X | - | - | - | - | - |
| PNP-β-D-galactosaminide | 0.1 | - | X | - | - | - | - |
| PNP-β-D-glucosaminide | 0.1 | - | - | X | - | - | - |
| 4-MU-α-D-glc | 0.1 | - | - | - | X | - | - |
| Y-phos.diNa | 0.1 | - | - | - | - | X | - |
| 3-indoxyl-choline-phosphate | 0.1 | - | - | - | - | - | X |

| | | | | | | | |
|---|---|---|---|---|---|---|---|
| Legend: H-Pro-AMC-HBr: L-proline-7-amido-4-methyl-coumarin-hydrobromide PNP-β-D-galactosaminide: 4-nitrophenyl-N-acetyl-β-D-galactosaminidine PNP-β-D-glucosaminide: 4-nitrophenyl-N-acetyl-β-D-glucosaminidine 4-MU-α-D-glc: 4-methyl-umbellipheryl-α-D-glucopyranoside Y-phos.diNa: 3-indoxyl-phosphate sodium salt | | | | | | | |

*Candida albicans* did not show satisfactory results in the present composition in the presence of the hexosaminidase substrates tested (PNP-β-D-galactosaminide and PNP-β-D-glucosaminide), as both of them diffuse to the medium and the chromogenic reaction cannot be associate to a determined colony.

The remaining *Candida* species did not decompose any of the substrates tested.

The compositions with pH values 6.4 and 6.8 promoted the greatest colony development observed (characteristic size and morphology) and deeper intensity of color.

### Example 21

The experiment aimed at assessing the effect of trehalose or glycerol addition on growth and differentiation by chromogenesis or fluorogenesis of *Candida* species. The composition tested and its variants are shown in table 30.

**Table 30. Composition of the nutritional medium to test diverse components**

| Components | g/L | 85 | 86 | 87 | 88 |
|---|---|---|---|---|---|
| Mixture of sweet potato extract and yeast extract | 20.0 | X | X | X | X |
| Monopotassium phosphate | 1.0 | X | X | X | X |
| Magnesium sulfate | 0.5 | X | X | X | X |
| Sodium deoxycholate | 0.5 | X | X | X | X |
| Glycerol | 10 (mL) | | | X | X |
| Trehalose | 10.0 | X | X | | |
| Nalidixic acid | 0.03 | X | X | X | X |
| Agar | 15.0 | X | X | X | X |
| X-β-D-glucosaminide | 0.1 | X | X | X | X |
| Salmon^{™}-β-D-glc | 0.1 | X | X | X | X |
| X-phos-inositol | 0.1 | X | | X | |
| 4-MU-α-D-glc | 0.1 | | X | | X |

Table 31 shows the results of these experiments.

The presence of glycerol favored the growth of some of the species tested *(Candida albicans, Candida krusei)*.

Trehalose, for some species, in the absence of glycerol, far from promoting growth, decreased it (for example, for *Candida krusei*).

The addition of trehalose did cause a significant intensification of the chromogenic reaction for *Candida albicans* and *Candida tropicalis.* This discovery was unexpected, as it had not been reported in the state-of-the-art papers, since trehalose has been broadly used in culture media to promote growth and to identify *Candida glabrata.*

*Candida albicans* is identified by its hexosaminidase (5-bromo-4-chloro-3-indoxyl-N-acetyl-β-D-glucosaminide), α-glucosidase (methyl-umbellipheryl-α-D-glucopyranoside) and phospholipase (ammonium salt of 5-bromo-4-chloro-3-indoxyl-mio-inositol-phosphate) activities; *Candida tropicalis* is identified by its α- and β-glucosidase (methyl-umbellipheryl-α-D-glucopyranoside and 6-chloro-3-indoxyl-β-D-glucopyranoside) and phospholipase (ammonium salt of 5-bromo-4-chloro-3-indoxyl-mio-inositol-phosphate) activities; *Candida kefyr* is identified by its β-glucosidase (6-chloro-3-indoxyl-β-D-glucopyranoside) activity; *Candida parapsilosis* is identified by its α-glucosidase (methyl-umbellipheryl-α-D-glucopyranoside) activity. *Candida krusei* and *Candida glabrata* by their white color (opaque or shiny, respectively) due to the absence of the aforementioned enzymatic activities.

**Table 31. Characteristic of diverse Candida species in the chromogenic and/or fluorogenic compositions**

| Microorganism | Variant | Characteristics of colonies | | | | | |
|---|---|---|---|---|---|---|---|
| | | Color | | | | Fluorescence | Morphology |
| | | Blue | Violet | Pink | White | | |
| C. albicans | 85 | X+ | - | - | - | - | Round, smooth, bulging, shiny, ∅ ≈ 2 mm |
| | 86 | X+ | - | - | - | X | |
| | 87 | X | - | - | - | - | |
| | 88 | X | - | - | - | X | |
| C. tropicalis | 85 | - | X+ | - | - | - | Round, smooth, bulging, shiny, ∅ ≈ 1-2 mm |
| | 86 | - | X | - | - | X | |
| | 87 | - | X | - | - | - | |
| | 88 | - | X | - | - | X | |
| C. glabrata | 85 | - | - | - | X | - | Round, smooth, bulging, shiny, ∅ ≤ 1 mm |
| | 86 | - | - | - | X | - | |
| | 87 | - | - | - | X | - | |
| | 88 | - | - | - | X | - | |
| C. kefyr | 85 | - | - | X | - | - | Round, smooth, bulging, shiny, ∅ ≤ 1 mm |
| | 86 | - | - | X | - | - | |
| | 87 | - | - | X | - | - | |
| | 88 | - | - | X | - | - | |
| C. krusei | 85 | - | - | - | X | - | Flattened, opaque, irregular edges, ∅ ≤ 2-4 mm variable |
| | 86 | - | - | - | X | - | |
| | 87 | - | - | - | X+ | - | |
| | 88 | - | - | - | X+ | - | |
| C. parapsilosis | 85 | - | - | - | X | - | Rough surface, regular edges |
| | 86 | - | - | - | X | X | |
| | 87 | - | - | - | X | - | |
| | 88 | - | - | - | X | X | |

| | | | | | | | |
|---|---|---|---|---|---|---|---|
| Legend: X+: High intensity of color | | | | | | | |

### Example 22

The composition for culture of fungus species is shown in table 32.

The pH of the composition was adjusted to 6.6.

As a result of these tests, a very marked differentiation of the fungus species of clinical interest was obtained. In particular, with variants 90 and 93, the intensity of the chromatic reactions was much greater than with any previously described in existing chromogenic culture media.

**Table 32. Nutritional medium to test maltose and trehalose addition in the presence of glycerol**

| Components | g/L | 89 | 90 | 91 | 92 | 93 |
|---|---|---|---|---|---|---|
| Sweet potato extract | Variable | 10.0 | 10.0 | 10.0 | 10.0 | 10.0 |
| Yeast extract | 10.0 | X | X | X | X | X |
| Monopotassium phosphate | 1.0 | X | X | X | X | X |
| Magnesium sulfate | 0.5 | X | X | X | X | X |
| Sodium deoxycholate | 0.5 | X | X | X | X | X |
| Glycerol | 10 (mL) | X | X | X | X | X |
| Trehalose | 10.0 | X | | | | |
| Maltose | 10.0 | | X | | | |
| Nalidixic acid | 0.03 | X | X | X | X | X |
| Agar | 15.0 | X | X | X | X | X |
| X-β-D-glucosaminide | 0.1 | X | X | X | X | X |
| Salmon^{™}-β-D-glc | 0.1 | X | X | X | X | X |
| X-phos-inositol | 0.1 | | | | X | |

In particular, *Candida albicans*, in the presence of trehalose or maltose combined with glycerol, unexpectedly showed a greenish blue or green color (for variant 90) very well differentiable from the rest of the species; *Candida tropicalis* took up an intense purplish blue color and, however, in variants 91 and 92, its colonies were bluish pink. *Candida kefyr* presented in all variants a pink coloration, while *Candida parapsilosis* was white in variants 89, 90 and 91, but took a pale blue coloration in variant 92. *Candida krusei* and *Candida glabrata*, which were white, could be identified by the opacity or brightness, respectively, as described in previous examples

Doubling the concentration of sweet potato extract favored intensity of the chromogenic reaction with *Candida albicans* and *Candida tropicalis*. In general, this improved the morphology (size) of the colonies of all species.

## Claims

1. Nutritional medium for cultivating yeasts **characterized by** its containing a mixture of sweet potato extract with yeast or tomato extract, agar and substances propitiating the identification of diverse *Candida* species selected between the chromogenic and fluorogenic substrates, indicators or colorants, carbon sources and organic and inorganic salts existing in the culture medium, selected among 5-bromo-4-chloro-3-indoxyl-mio-inositol-phosphate, methyl-umbellipheryl-α-D-glucopyranoside, 5-bromo-4-chloro-3-indolyl-N-acetyl-β-D-glucopyranoside, 6-chloro-3-indoxyl-β-D-glucopyranoside, 4-methyl-umbellipheryl-N-acetyl-β-D-galactosaminide, 5-bromo-4-chloro-3-indoxyl-N-acetyl-β-D-glucosaminide, L-proline-7-amido-4-methyl-coumarin hydrobromide, L-proline paranitroanilide, bromocresol purple, bromothymol blue, skim milk, glycerol and magnesium sulfate.

2. Nutritional medium for cultivate yeasts according to claim 1 **characterized by** its containing a mixture of sweet potato extract with yeast or tomato extract in quantity of 20 to 30 g/L, where the sweet potato extract is present in quantity of 50 to 67 % with regard to the mass of mixture, from 13 to 15 g/L agar and substances propitiating identification of the diverse *Candida* species, selected among chromogenic and fluorogenic substrates, indicators or colorants, carbon sources and organic and inorganic salts existing in the culture medium in quantity from 0 to 26 g/L, preferably 5-bromo-4-chloro-3-indoxyl-mio-inositol-phosphate in quantities from 0 to 0.1 g/L, methyl-umbellipheryl-α-D-glucopyranoside from 0 to 0.1 g/L, 5-bromo-4-chloro-3-indolyl-N-acetyl-β-D-glucopyranoside from 0 to 0.1 g/L, 6-chloro-3-indoxyl-β-D-glucopyranoside from 0 to 0.1 g/L, 4-methyl-umbellipheryl-N-acetyl-β-D-galactosaminide from 0 to 0.15 g/L, 5-bromo-4-chloro-3-indoxyl-N-acetyl-β-D-glucosaminide from 0 to 0.1 g/L, L-proline-7-amido-4-methyl-coumarin hydrobromide from 0 to 0.5 g/L, L-proline paranitroanilide from 0 to 0.2 g/L, bromocresol purple from 0 to 0.04 g/L, bromothymol blue from 0 to 0.08 g/L, skim milk from 0 to 5 g/L, glycerol from 0 to 25 g (mL)/L and magnesium sulfate from 0 to 0.5 g/L.

3. Nutritional medium according to claims 1 and 2, **characterized by** its containing a combination of chromogenic-reaction activators consisting in maltose or trehalose.

4. Nutritional medium according to claim 3, **characterized by** its contents of maltose or trehalose in quantities of 1 g per g (mL) of glycerol.

5. Nutritional medium according to claims 1 to 4, **characterized by** its containing substances supplying nitrogen, specifically those selected among glycine, bacteriological peptone and enzymatic casein hydrolysate.

6. Nutritional medium according to claim 5, **characterized by** its contents of substances supplying nitrogen in quantities from 0 to 13 g/L, specifically glycine at a concentration ranging from 0 to 3 g/L, bacteriological peptone from 0 to 5 g/L and enzymatic casein hydrolysate from 0 to 5 g/L.

7. Nutritional medium according to claims 1 to 6, **characterized by** its containing bacteria inhibitors preferably selected among nalidixic acid, rose bengal, sodium deoxycholate, sodium sulfite, ammonium-bismuth citrate and tetraphenyltetrazolium chloride.

8. Nutritional medium according to claim 7, **characterized by** its contents of bacteria inhibitors in quantities from 0 to 10 g/L, more specifically nalidixic acid added in quantity from 0 to 0.05 g/L, rose bengal in quantity from 0 to 0.05 g/L, sodium deoxycholate from 0 to 0.5 g/L, sodium sulfite from 0 to 3 g/L, ammonium-bismuth citrate from 0 to 5 g/L and tetraphenyltetrazolium chloride from 0 to 0.05 g/L.

9. Nutritional medium according to claims 1 to 8, **characterized by** its containing pH regulating substances and enzyme activators in quantities from 0 to 6 g/L, preferably monopotassium phosphate, dipotassium phosphate and 3-Morpholino-propansulfonsaure.

10. Nutritional medium according to claim 9, **characterized by** its contents of monopotassium phosphate in a quantity from 0 to 1 g/L, dipotassium phosphate from 0 to 4 g/L and 3-Morpholino-propansulfonsaure (3-morpholine-propane sulfonic acid), also known as MOPS, from 0 to 0.5 g/L.

11. Nutritional medium according to claims 1 to 10, **characterized by** the entirety of the ingredients to be used having been added in quantities from 35 to 50 g in 1 liter of distilled or deionized water in the pH range from 5 to 7.

12. Nutritional medium according to claim 11, **characterized by** its pH having been adjusted from 6.0 to 6.8.

## Patentansprüche

1. Nährmedium für die Zucht von Hefen, **dadurch gekennzeichnet, dass** es eine Mischung von Süsskartoffelextrakt mit Hefe oder Tomatenextrakt, Agar und Substanzen enthält, die die Identifizierung von verschiedenen Arten von *Candida* begünstigen, ausgewählt unter chromogenen und fluorogenen Substraten, Indikatoren oder Farbstoffen, Kohlenstoffquellen und organischen und anorganischen Salzen, die im Kuturmittel vorhanden sind, die unter 5-Brom-4-chlor-4-indoxyl-mio-inositol-phosphat, Methylumbelliphenyl-α-D-glucopyranosid, 5-Brom-4-chlor-3-3-indolyl-N-acetyl-β-D-glucopyranosid, 6-Chlor-3-indoxyl-β-D-glucopyranosid, 4-methylumbelliphenyl-N-acetyl-β-D-galactosaminid, 5-Brom-4-chlor-3-indoxyl-N-acetyl-β-D-glucosaminid, L-Prolin-7-amido-4-methyl-coumarin Hydrobromid, L-Prolin Paranitroanilid, Bromcresol Purpur, Bromthymol Blau, Magermilch und Magnesium Sulfat ausgewählt werden.

2. Nährmedium für die Zucht von Hefen gemäss Anspruch 1 **gekennzeichnet dadurch, dass** es eine Mischung von Süsskartoffelextrakt mit Hefe oder Tomatenextrakt in einer Menge von 20 bis 30 g/l enthält, in der der Süsskartoffelextrakt in einer Menge von 50 bis 67% der gesamten Menge der Mischung vorhanden ist, 13 bis 15 g/l Agar und Substanzen, die die Identifizierung von verschiedenen Arten von *Candida* begünstigen, ausgewählt unter chromogenen und fluorogenen Substanzen, Indikatoren oder Farbstoffen, Kohlenstoffquellen und organischen und anorganischen Salzen, die im Kulturmedium in einer Menge von 0 bis 26 g/l vorhanden sind, bevorzugt 5-Brom-4.chlor-3-indoxyl-mio-inositol-phosphat in einer Menge von 0 bis 0,1 g/l, Methyl-umbelliphenyl-α-D-glucopyranosid in einer Menge von 0 bis 0,1 g/l, 6-Chlor-3-indoxyl-β-D-glucopyranosid in einer Menge von 0 bis 0,1 g/l, 4-Methyl-umbelliphenyl-N-acetyl-β-D-galactosaminid in einer Menge von 0 bis 0,15 g/l, 5-Brom-4-chlor3-indoxyl-N-acetyl-β-D-glucosaminid in einer Menge von 0 bis 0,1 g/l, L-Prolin-7-amido-4-methyl-coumarin Hydrobromid in einer Menge von 0 bis 0,5 g/l, L-Prolin Paranitroanilid in einer Menge von 0 bis 0,2 g/l, Promocresol Purpur in einer Menge von 0 bis 0,04 g/l, Bromthymol Blau in einer Menge von 0 bis 0,08 g/l, Magermilch in einer Menge von 0 bis 5 g/l, Glycerol in einer Menge von 0 bis 25 g (mL)/l und Magnesiumsulfat in einer Menge von 0 bis 0,5 g/l.

3. Nährmedium gemäss Anspruch 1 und 2 **gekennzeichnet dadurch, dass** es eine Kombination von Aktivatoren der chromogenen Reaktion enthält, die aus Maltose oder Trehalose bestehen.

4. Nährmedium gemäss Anspruch 3 **gekennzeichnet dadurch, dass** die Maltose oder Trehalose in Mengen von 1 g pro g (ml) Glycerol vorhanden ist.

5. Nährmedium gemäss Anspruch 1 bis 4 **gekennzeichnet dadurch, dass** es Substanzen enthält, die Stickstoff liefern, besonders solche, die unter Glycin, bakteriologischem Pepton und enzymatischem Caseinhydrolysat ausgewählt wurden.

6. Nährmedium gemäss Anspruch 5 **gekennzeichnet dadurch, dass** es Substanzen, die Stickstoff liefern, in Mengen von 0 bis 13 g/l, spezifisch Glycin in einer Konzentration von 0 bis 3 g/l, bakteriologisches Pepton von 0 bis 5 g/l und enzymatisches Caseinhidrolysat von 0 bis 5 g/l enthält.

7. Nährmedium gemäss Anspruch 1 bis 6 **gekennzeichnet dadurch, dass** es Bakterieninhibitoren enthält, die bevorzugt unter Nalidixic Säure, Bengalrosa, Natriumdeoxycholat, Natriumsulfit, Ammonium-bismuth-citrat und Tetraphenyltetrazoliumchlorid ausgewählt werden.

8. Nährmedium gemäss Anspruch 7 **gekennzeichnet dadurch, dass** es Mengen von Bakterieninhibitoren von 0 bis 10 g/l enthält, noch spezifischer Nalidixic Säure hinzugefügt in einer Menge von 0 bis 0,05 g/l, Bengalrosa in einer Menge von 0 bis 0,05 g/l, Natriumdeoxicholat in einer Menge von 0 bis 0,5 g/l, Natriumsulfit in einer Menge von 0 bis 3 g/l, Ammonium-bismuth-citrat in einer Menge von 0 bis 5 g/l und Tetraphenyltetrazolium-chlorid in einer Menge von 0 bis 0,05 g/l.

9. Nährmedium gemäss Anspruch 1 bis 8 **gekennzeichnet dadurch, dass** es pH-Regulatorsubstanzen und Enzymaktivatoren in Mengen von 0 bis 6 g/l enthält, bevorzugt Monokalium-phosphat, Dikalium-phosphat und 3-Morpholino-propansulfonsaure.

10. Nährmedium gemäss Anspruch 9 **gekennzeichnet dadurch, dass** es Monokalium Phosphat in einer Menge von 0 bis 1 g/l, Dikalium Phosphat in einer Menge von 0 bis 4 g/l und 3-Morpholino-propansulfonsaure (3-Morpholin-propane Sulfonsäure) auch bekannt als MOPS in einer Menge von 0 bis 0,5 g/l enthält.

11. Nährmedium gemäss Anspruch 1 bis 10 **gekennzeichnet dadurch, dass** die gesamten verwendeten Bestandteile in Mengen von 35 bis 50 g in 1 Liter destilliertem oder deinosiertem Wasser in einem pH-Bereich von 5 bis 7 hinzugefügt wurden.

12. Nährmedium gemäss Anspruch 11 **gekennzeichnet dadurch, dass** der pH-Wert auf 6,0 bis 6,8 eingestellt wurde.

## Revendications

1. Milieu nutritif pour cultiver des levures, **caractérisé en ce qu'**il contient un mélange d'extrait de patate douce avec de la levure ou un extrait de tomate, de la gélose et des substances qui favorisent l'identification de diverses espèces de *Candida,* choisi parmi les substrats chromogéniques et fluorogéniques, les indicateurs ou les colorants, les sources de carbone et les sels organiques et inorganiques existant dans le milieu de culture, choisi parmi le 5-bromo-4-chloro-3-indoxyl-mio-inositol-phosphate, le méthylumbélliphéryl-α-D-glucopyranoside, le 5-bromo-4-chloro-3-indolyl-N-acétyl-β-D-glucopyranoside, le 6-chloro-3-indoxyl-β-D-glucopyranoside, le 4-méthylumbelliphéryl-N-acétyl-β-D-galactosaminide, le 5-bromo-4-chloro-3-indoxyl-N-acétyl-β-D-glucosaminide, le L-proline-7-amido-4-méthyl-coumarin hydrobromure, le L-proline paranitroanilide, le violet de bromocrésol, le bleu de bromothymol, le lait écrémé, le glycérol et le sulfate de magnésium.

2. Milieu nutritif pour cultiver des levures selon la revendication 1, **caractérisé en ce qu'**il contient un mélange d'extrait de patate douce avec de la levure ou un extrait de tomate en une quantité de 20 a 30 g/l, dans lequel l'extrait de patate douce est présent en une quantité de 50 à 67 % par rapport à la masse du mélange, de 13 à 15 g/l de gélose et des substances qui favorisent l'identification des diverses espèces de *Candida,* choisi parmi des substrats chromogéniques et fluorogéniques, des indicateurs ou des colorants, des sources de carbone et des sels organiques et inorganiques existant dans le milieu de culture en une quantité de 0 à 26 g/l, de préférence le 5-bromo-4-chloro-3-indoxyl-mio-inositol-phosphate en des quantités de 0 à 0,1 g/l, le méthylumbélliphéryl-α-D-glucopyranoside de 0 à 0,1 g/l, le 5-bromo-4-chloro-3-indolyl-N-acétyl-β-D-glucopyranoside de 0 à 0,1 g/l, le 6-chloro-3-indoxyl-β-D-glucopyranoside de 0 à 0,1 g/l, le 4-méthylumbelliphéryl-N-acétyl-β-D-galactosaminide de 0 à 0,15 g/l, le 5-bromo-4-chloro-3-indoxyl-N-acétyl-β-D-glucosaminide de 0 à 0,1 g/l, le L-proline-7-amido-4-méthyl-coumarin hydrobromure de 0 à 0,5 g/l, le L-proline paranitroanilide de 0 à 0,2 g/l, le violet de bromocrésol de 0 à 0,04 g/l, le bleu de bromothymol de 0 à 0,08 g/l, le lait écrémé de 0 à 5 g/l, le glycérol de 0 à 25 g/ml)/l et le sulfate de magnésium de 0 à 0,5 g/l.

3. Milieu nutritif selon les revendications 1 et 2, **caractérisé en ce qu'**il contient une combinaison d'activateurs de réaction chromogénique consistant en du maltose ou du tréhalose.

4. Milieu nutritif selon la revendication 3, **caractérisé en ce qu'**il contient du maltose ou du tréhalose en des quantités de 1 g par g (ml) de glycérol.

5. Milieu nutritif selon les revendications 1 à 4, **caractérisé en ce qu'**il contient des substances qui fournissent de l'azote, particulièrement celles choisies parmi la glycine, la peptone bactériologique et l'hydrolysat enzymatique de caséine.

6. Milieu nutritif selon la revendication 5, **caractérisé en ce qu'**il contient des substances qui fournissent de l'azote en des quantités de 0 à 13 g/l, particulièrement de la glycine à une concentration variant entre 0 et 3 g/l, de la peptone bactériologique de 0 à 5 g/l et de l'hydrolysat enzymatique de caséine de 0 à 5 g/l.

7. Milieu nutritif selon les revendications 1 à 6, **caractérisé en ce qu'**il contient des inhibiteurs de bactéries choisis de préférence entre l'acide nalidixique, rose bengale, desoxycholate de sodium, sulfite de sodium, citrate d'ammonium de bismuth et chlorure de tétraphényltétrazolium.

8. Milieu nutritif selon la revendication 7, **caractérisé en ce qu'**il contient des inhibiteurs de bactéries en des quantités de 0 à 10 g/l, particulièrement de l'acide nalidixique en des quantités de 0 à 0,05 g/l, du rose bengale en des quantités de 0 à 0,05 g/l, du desoxycholate de sodium de 0 à 0,5 g/l, du sulfite de sodium de 0 à 3 g/l, du citrate d'ammonium de bismuth de 0 à 5 g/l et du chlorure de tétraphényltétrazolium de 0 à 0,05 g/l.

9. Milieu nutritif selon les revendications 1 à 8, **caractérisé en ce qu'**il contient des substances régulatrices de pH y des activateurs enzymatiques en des quantités de 0 à 6 g/l, de préférence du phosphate de monopotassium, du phosphate de dipotassium et du 3-Morpholino-propansulfonsaure.

10. Milieu nutritif selon la revendication 9, **caractérisé en ce qu'**il contient du phosphate de monopotassium en une quantité de 0 à 1 g/l, du phosphate de dipotassium de 0 à 4 g/l et du 3-Morpholino-propansulfonsaure (acide 3-morpholine-propane sulfonique), connu aussi sous le nom de MOPS, de 0 à 0,5 g/l.

11. Milieu nutritif selon les revendications 1 à 10, **caractérisé en ce que** la totalité des ingrédients qui doivent être utilisés a été ajoutée en des quantités allant de 35 à 50 g dans 1 litre d'eau distillée ou déionisée sur la gamme de pH de 5 à 7.

12. Milieu nutritif selon la revendication 11, **caractérisé en ce que** son pH a été ajusté entre 6,0 et 6,8.
